(19) Europäisches Patentamt
European Patent Office
Office européen des brevets

(11) EP 1 789 014 B1

(12) **EUROPÄISCHE PATENTSCHRIFT**

(45) Veröffentlichungstag und Bekanntmachung des Hinweises auf die Patenterteilung:
**27.02.2008 Patentblatt 2008/09**

(21) Anmeldenummer: **05771860.3**

(22) Anmeldetag: **18.08.2005**

(51) Int Cl.:
***A61Q 5/10*** (2006.01) ***A61K 8/49*** (2006.01)
***A61K 8/33*** (2006.01) ***C07D 239/36*** (2006.01)

(86) Internationale Anmeldenummer:
**PCT/EP2005/008950**

(87) Internationale Veröffentlichungsnummer:
**WO 2006/029687 (23.03.2006 Gazette 2006/12)**

(54) **MITTEL ZUM FÄRBEN VON KERATINHALTIGEN FASERN**

SUBSTANCE FOR DYEING KERATINIC FIBERS

SUBSTANCES SERVANT A TEINDRE DES FIBRES KERATINIQUES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **14.09.2004 DE 102004044231**

(43) Veröffentlichungstag der Anmeldung:
**30.05.2007 Patentblatt 2007/22**

(73) Patentinhaber: **HENKEL KOMMANDITGESELLSCHAFT AUF AKTIEN**
**40589 Düsseldorf (DE)**

(72) Erfinder:
- **GROSS, Wibke**
  **40549 Düsseldorf (DE)**
- **MAUSBERG, Sandra**
  **40699 Erkrath (DE)**
- **OBERKOBUSCH, Doris**
  **40591 Düsseldorf (DE)**
- **HÖFFKES, Horst**
  **40595 Düsseldorf (DE)**

(56) Entgegenhaltungen:
**WO-A-20/04022016** **FR-A- 2 787 707**

EP 1 789 014 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft ein Mittel zum Färben von keratinhaltigen Fasern, insbesondere menschlichen Haaren, das spezielle in 1-Stellung substituierte 1,2-Dihydro-3,4,6-trimethyl-2-oxo-pyrimidinium-Derivate in Kombination mit ausgewählten Aldehyden als reaktive Carbonylverbindung enthält, die Verwendung dieser Kombination in Mitteln zum Färben von keratinhaltigen Fasern, zur Farbauffrischung bzw. Nuancierung von bereits gefärbten keratinhaltigen Fasern sowie ein Verfahren zum Färben von keratinhaltigen Fasern, insbesondere menschlichen Haaren.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridin-derivate verwendet. Als Kupplersubstanzen eignen sich insbesondere $\alpha$-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. $H_2O_2$, was in einigen Fällen Schädigungen der Faser zur Folge haben kann. Problematisch gestaltet sich nach wie vor eine Bereitstellung von Oxidationshaarfärbungen im Rotbereich mit ausreichenden Echtheitseigenschaften, insbesondere in mit sehr guten Wasch- und Reibechtheiten.

Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

In der Veröffentlichung H. Baumann et al., Liebigs Ann. Chem., 1968, 717, 124-136, werden Reaktionen von Pyrimidonen als Methylenbasen beschrieben. Ein Haarfärbemittel, enthaltend 1,2-Dihydro-2-oxo-pyrimidinium-Derivate, oder die Verwendung der offenbarten Hemicyanine zum Färben von keratinhaltigen Fasern wird hier nicht vorgeschlagen.

In der deutschen Patentanmeldung DE-A1-2047431 werden kationische Methinfarbstoffe zur Färbung von anionisch modifizierten Fasern, wie sauer modifizierten Polyestern oder Acrylnitrilpolymerisaten beschrieben. Zur Darstellung der kationischen Methinfarbstoffe werden unter anderem 3,4-Dihydro-3-methyl-4-methylenchinazol-2-on und 1,3,6-Trimethyl-4-methylen-pyrimidin-2-on sowie zwingend Terephthalaldehyd verwendet.

In der deutschen Patentanmeldung DE-A1-2165913 wird ein Verfahren zur Herstellung von Ausbleichbildern unter Verwendung von lichtempfindlichen Farbstoffen vorgeschlagen. Die beanspruchten lichtempfindlichen Farbstoffe gehören zu der Klasse der Pyrimidon- bzw. Thiopyrimidonfarbstoffe.

In der deutschen Patentanmeldung DE-A1-102 41 076 werden 1,2-Dihydro-2-oxopyrimidinium-Derivate in Kombination mit reaktiven Carbonylverbindungen als Mittel zur Färbung von Keratinfasern vorgeschlagen. Allerdings sind diese Mittel noch verbesserungswürdig im Hinblick auf die Echtheit der damit erzielten Färbungen, insbesondere der Licht- und Waschechtheit.

In Kenntnis der Druckschrift DE-A1-2165913 ist es bekannt, dass die Farbstoffe auf Basis der Pyrimidone lichtempfindlich sind. Zwar wurde in der DE-A1-102 41 076 gefunden, dass sich diese Farbstoffe zur Haarfärbung auch in Bezug auf die Lichtechtheiten gut eignen, allerdings war gerade der Parameter der Lichtechtheit noch verbesserungswürdig. Aufgabe der vorliegenden Erfindung ist es somit, Färbemittel für keratinhaltige Fasern, insbesondere menschliche Haare, bereitzustellen, die hinsichtlich der Farbtiefe, der Grauabdeckung und den Echtheitseigenschaften, wie beispielsweise Licht-, Reib- und Waschechtheit sowie Schweiß- und Kaltwellechtheit, insbesondere der Wasch- und Lichtechtheit, qualitativ den Farbstoffen gemäß DE-A1-102 41 076 überlegen sind. Darüber hinaus dürfen die Färbemittel kein oder lediglich ein sehr geringes Sensibilisierungspotential aufweisen.

Überraschenderweise wurde nun gefunden, daß die in der Formel I dargestellten Verbindungen in Kombination mit ausgewählten Aldehydderivaten, sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Es werden auch ohne den Einsatz von Oxidationsmitteln insbesondere Ausfärbungen mit verbesserten Wasch- und Lichtechtheitseigenschaften über einen Nuancenbereich von gelb über gelbbraun, orange, braunorange, braun, rot, rotviolett bis hin zu blauviolett, dunkelblau und schwarz erhalten. Der Einsatz von oxidierenden Agentien soll jedoch nicht prinzipiell ausgeschlossen werden.

Ein erster Gegenstand der Erfindung ist ein Mittel zum Färben von keratinhaltigen Fasern, insbesondere menschlichen Haaren, enthaltend als Komponente A mindestens eine Verbindung gemäß Formel I und/oder deren Enaminform,

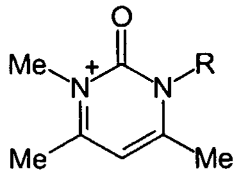

$X^-$ (I)

wobei

- R für eine Allylgruppe, eine Hydroxy-($C_2$- bis $C_6$)-alkylgruppe oder eine gegebenenfalls substituierte Benzylgruppe steht,
- $X^-$ ein physiologisch verträgliches Anion bedeutet

und als Komponente B mindestens einen Aldehyd ausgewählt aus der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd und 4-Dimethylamino-2-methoxybenzaldehyd.

Beispiele für Hydroxy-($C_2$-bis $C_6$)-alkylgruppen der Formel (I) sind 2-Hydroxyethyl, 2-Hydroxypropyl, 3-Hydroxypropyl, 4-Hydroxybutyl, und 6-Hydroxyhexyl. Bevorzugte Hydroxy-($C_2$-bis $C_6$)-alkylgruppen gemäß Formel (I) sind 2-Hydroxyethyl, 2-Hydroxypropyl und 3-Hydroxypropyl, besonders bevorzugt sind 2-Hydroxypropyl und 3-Hydroxypropyl.

R gemäß Formel (I) steht bevorzugt für eine Allylgruppe, eine 3-Hydroxypropylgruppe, eine 2-Hydroxypropylgruppe, eine 2-Hydroxyethylgruppe oder eine Benzylgruppe.

Es ist bevorzugt, wenn $X^-$ gemäß Formel (I) ausgewählt wird aus Halogenid, Benzolsulfonat, p-Toluolsulfonat, ($C_1$- bis $C_4$)-Alkansulfonat, Trifluormethansulfonat, Perchlorat, 0.5 Sulfat, Hydrogensulfat, Tetrafluoroborat, Hexafluorophosphat oder Tetrachlorozinkat. Besonders bevorzugt steht $X^-$ für Chlorid, Bromid oder Hydrogensulfat.

Die Nummerierung der ringbildenden Atome des Heterozyklus wird in nachfolgender Formel (II-1) offenbart.

Vorzugsweise werden die Verbindungen gemäß Formel I ausgewählt aus der Gruppe, bestehend aus den physiologisch verträglichen Salzen

des 1-Allyl-1,2-dihydro-3,4,6-trimethyl-2-oxo-pyrimidiniums, des 1,2-Dihydro-1-(2-hydroxyethyl)-3,4,6-trimethyl-2-oxopyrimidiniums, des 1,2-Dihydro-1-(3-hydroxypropyl)-3,4,6-trimethyl-2-oxopyrimidiniums und des 1-Benzyl-1,2-dihydro-3,4,6-trimethyl-2-oxo-pyrimidiniums und
den Enaminformen
des 1-Allyl-1,2-dihydro-3,4,6-trimethyl-2-oxo-pyrimidiniums, des 1,2-Dihydro-1-(2-hydroxyethyl)-3,4,6-trimethyl-2-oxopyrimidiniums, des 1,2-Dihydro-1-(3-hydroxypropyl)-3,4,6-trimethyl-2-oxopyrimidiniums und des 1-Benzyl-1,2-dihydro-3,4,6-trimethyl-2-oxo-pyrimidiniums.

Bei den o.g. Salzen ist es wiederum bevorzugt, das Gegenion auszuwählen aus Halogenid, Benzolsulfonat,

p-Toluolsulfonat, ($C_1$- bis $C_4$)-Alkansulfonat, Trifluormethansulfonat, Perchlorat, 0.5 Sulfat, Hydrogensulfat, Tetrafluoroborat, Hexafluorophosphat oder Tetrachlorozinkat, insbesondere aus Bromid, Chlorid, Hydrogensulfat und p-Toluolsulfonat.

Besonders bevorzugt werden die Verbindungen der Formel (I) ausgewählt aus der Gruppe bestehend aus den Verbindungen gemäß Formeln (II-1) bis (II-12), nämlich den Bromiden, Chloriden oder Hydrogensulfaten des 1-Allyl-1,2-dihydro-3,4,6-trimethyl-2-oxo-pyrimidiniums, des 1,2-Dihydro-1-(2-hydroxyethyl)-3,4,6-trimethyl-2-oxopyrimidiniums, des 1,2-Dihydro-1-(3-hydroxypropyl)-3,4,6-trimethyl-2-oxopyrimidiniums und des 1-Benzyl-1,2-dihydro-3,4,6-trimethyl-2-oxo-pyrimidiniums sowie den Enaminformen dieser Verbindungen. Die Verbindungen werden erschöpfend nachfolgend in den Formeln (II-1) bis (II-12) offenbart:

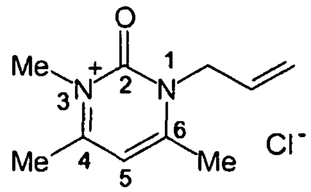

(II-1)

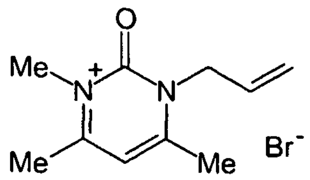

(II-2)

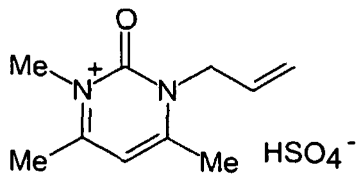

(II-3)

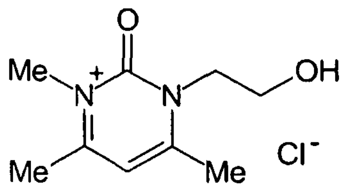

(II-4)

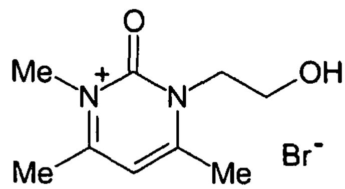

(II-5)

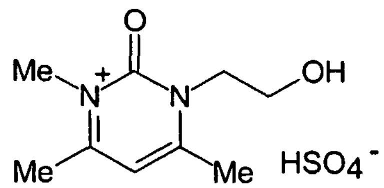

(II-6)

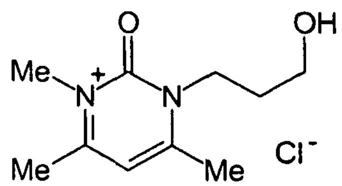

(II-7)

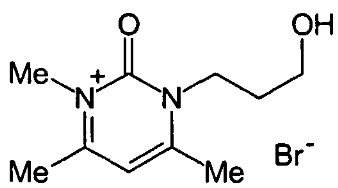

(II-8)

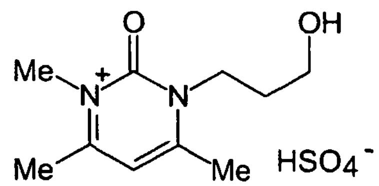

(II-9)

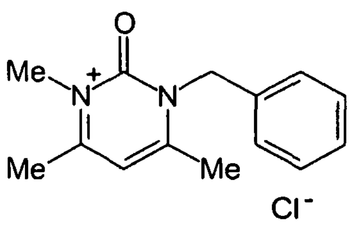

(II-10)

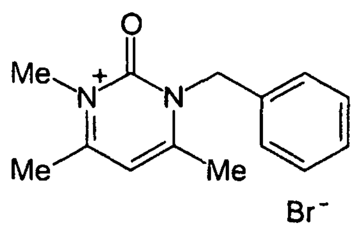

(II-11)

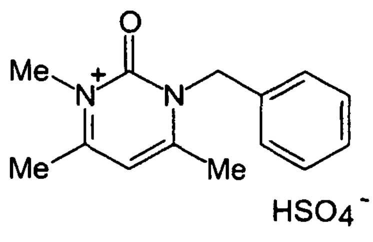

(II-12)

Als CH-acide Verbindungen werden im Allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Die erfindungsgemäßen Verbindungen gemäß Formel I und Formeln (II-1) bis (II-12) sind CH-acide Verbindungen.
Sie liegen im chemischen Gleichgewicht mit ihrer korrespondierenden Enaminform vor. Mit Hilfe einer Base lassen sich aus den Verbindungen der besagten Formeln durch Deprotonierung am Kohlenstoffatom der aktivierten Methylgruppen in 4- oder 6-Position die entsprechenden Enamine gezielt darstellen. Exemplarisch wird diese Deprotonierung nachfolgend illustriert. Verbindungen gemäß Formeln Ia bzw. Ib sind Beispiele für die erfindungsgemäßen Enaminformen der 1,2-Dihydro-3,4,6-trimethyl-2-oxo-pyrimidinium-Derivate.

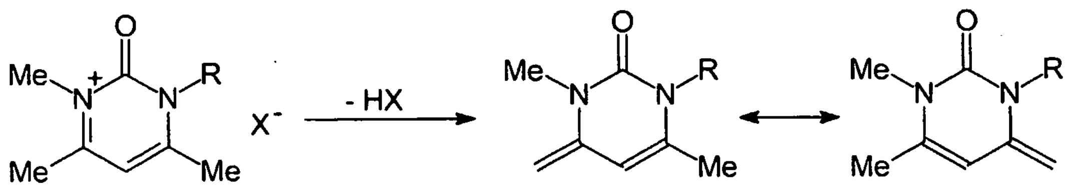


(I) (Ia) (Ib)

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose verwendet werden.

1,2-Dihydro-2-oxo-pyrimidinium-Derivate sind allgemein literaturbekannt oder im Handel erhältlich. Die erfindungsgemäßen Verbindungen der Formel I sind neu, aber nach bekannten Syntheseverfahren nach D. Lloyd et al., J. Chem. Soc. Perkin Trans I, 1977, 16, 1862-1869; S. T. Oswald et al., J. Heterocycl. Chem., 1974, 11(3), 441-443; H. Baumann et al., Liebigs Ann. Chem., 1968, 717, 124-136 und V. A. Chuiguk, Ukr. Khim. Zh. (Russ Ed.), 1982, 48(11), 1220-1223 herstellbar.

In einer zweiten Ausführungsform kann es zur Erweiterung des Farbspektrums vorteilhaft sein, den erfindungsgemäßen Mitteln neben mindestens einer Verbindung gemäß Formel (I) als Komponente A und mindestens einer Verbindung der Komponente B mindestens eine weitere Verbindung als Komponente C zuzusetzen. Die Verbindung der Komponente C wird ausgewählt aus (a) CH-aciden Verbindungen, welche von Verbindungen der Formel (I) verschiedenen sind, und/oder (b) reaktiven Carbonylverbindungen, welche von den Verbindungen der Komponente B verschieden sind.

Reaktive Carbonylverbindungen als zusätzliche Komponente C besitzen im Sinne der Erfindung mindestens eine Carbonylgruppe als reaktive Gruppe, welche mit der CH-aciden Verbindung gemäß Formel I unter Ausbildung einer Kohlenstoff-Kohlenstoff-Bindung reagiert. Ferner sind erfindungsgemäß auch solche Verbindungen als Komponente C verwendbar, in denen die reaktive Carbonylgruppe derart derivatisiert bzw. maskiert ist, daß die Reaktivität des Kohlenstoffatoms der derivatisierten Carbonylgruppe gegenüber den CH-aciden Verbindungen der Formel I stets vorhanden ist. Diese Derivate sind bevorzugt Additionsverbindungen

a) von Aminen und deren Derivate unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente B leitet sich in diesem Fall c) von einem Aldehyd ab)

an das Kohlenstoffatom der Carbonylgruppe der reaktiven Carbonylverbindung.

Die zusätzlichen CH-aciden Verbindungen der Komponente C sind bevorzugt ausgewählt aus der Gruppe bestehend aus mit physiologisch verträglichen Anionen, insbesondere p-Toluolsulfonaten, Methansulfonaten, Hydrogensulfaten, Tetrafluoroboraten und Halogeniden, wie den Chloriden, Bromiden und Iodiden, gebildeten Salzen des 1,4-Dimethylchinoliniums, 1-Ethyl-4-methyl-chinoliniums, 1-Ethyl-2-methylchinoliniums, 1,2,3,3-Tetramethyl-3H-indoliums, 2,3-Dimethyl-benzothiazoliums, 2,3-Dimethyl-naphtho[1,2-d]thiazoliums, 3-Ethyl-2-methyl-naphtho[1,2-d]thiazoliums, 3-Ethyl-2-methyl-benzoxazoliums, 1,2,3-Trimethylchinoxaliniums, 3-Ethyl-2-methyl-benzothiazoliums, 1,2-Dihydro-1,3,4,6-tetramethyl-2-oxo-pyrimidiniums, 1,2-Dihydro-1,3,4-trimethyl-2-oxo-pyrimidiniums, 1,2-Dihydro-4,6-dimethyl-1,3-dipropyl-2-oxopyrimidiniums, 1,2-Dihydro-1,3,4,6-tetramethyl-2-thioxo-pyrimidiniums, 1,2-Dihydro-1,3,4,5,6-pentamethyl-2-oxo-pyrimidiniums, 2,5-Dimethyl-3-(2-propenyl)-1,3,4-thiadiazoliums, 3-Ethyl-2,5-dimethyl-1,3,4-thiadiazoliums, 1,2-Dimethylchinoliniums und 1,3,3-Trimethyl-2-methylenindolin (Fischersche Base), Oxindol, 3-Methyl-1-phenyl-pyrazolin-5-on, Indan-1,2-dion, Indan-1,3-dion, Indan-1-on, 2-Amino-4-imino-1,3-thiazolinhydrochlorid, Benzoylacetonitril, 3-Dicyanmethylenindan-1-on, 2-(2-Furanoyl)acetonitril, 2-(2-Theonyl)acetonitril, 2-(Cyanmethyl)benzimidazol, 2-(Cyanmethyl)-benzothiazol und 2-(2,5-Dimethyl-3-furanoyl)acetonitril.

Bevorzugte zusätzliche reaktive Carbonylverbindungen der Komponente C werden ausgewählt aus der Gruppe, bestehend aus Benzaldehyd und seinen Derivaten, Naphthaldehyd und seinen Derivaten, Zimtaldehyd und seinen Derivaten, 2,3,6,7-Tetrahydro-1H,5H-benzo[ij]chinolizin-9-carboxaldehyd, 2,3,6,7-Tetrahydro-8-hydroxy-1 H,5H-benzo [ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 2-(1',3',3'-Trimethyl-2-indolinyliden)-acetaldehyd, 1-Methylpyrrol-2-aldehyd, 4-Pyridinaldehyd, 2-Pyridinaldehyd, 3-Pyridinaldehyd, Pyridoxal, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 5-(4-Dimethylamino-1-naphthyl)penta-3,5-dienal, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Formyl-1-methylpyridinium-, 2-Formyl-1-methylpyridinium-, 4-Formyl-1-ethylpyridinium- ,2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-benzolsulfonat, -p-toluolsulfonat, -methansulfonat, - perchlorat, -sulfat, -chlorid, -bromid, -iodid, -tetrachlorozinkat, -methylsulfat-, trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Die Derivate der Benzaldehyde, Naphthaldehyde bzw. Zimtaldehyde der reaktiven Carbonylverbindung gemäß Komponente C werden bevorzugt ausgewählt aus Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxybenzaldehyd, 2-Hydroxy-4-methoxybenzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methylbenzaldehyd, 2,4-Dihydroxy-5-methyl-benzaldehyd, 2,4-Dihydroxy-6-methylbenzaldehyd, 2,4-Dihydroxy-3-methoxy-benzaldehyd, 2,4-Dihydroxy-5-methoxybenzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methylbenzaldehyd, 3,4-Dihydroxy-5-methyl-benzaldehyd, 3,4-Dihydroxy-6-methylbenzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzatdehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd,

6-Nitropiperonal, 2-Nitropiperonal, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd, 4-Diphenylamino-benzaldehyd, 4-(1-Imidazolyl)-benzaldehyd und Piperonal. Diese Vertreter sind zugleich die besonders bevorzugten zusätzlichen reaktiven Carbonylverbindungen der Komponente C.

Aus den Druckschriften EP-A2-998 908 und JP-A2-2002047153 sind dem Fachmann Haarfärbemittel bekannt, die u.a. als direktziehenden Farbstoff mindestens eine Verbindung mit einem 1,2-Dihydro-2-oxopyrimidinium-Rest enthalten. In einer dritten Ausführungsform enthält das Färbemittel zusätzlich mindestens ein Reaktionsprodukt (im folgenden als Reaktionsprodukt RP bezeichnet) aus einem 1,2-Dihydro-2-oxopyrimidinium-Derivat der Formel I und einer Verbindung der Komponente B als direktziehenden Farbstoff. Derartige Reaktionsprodukte RP können z. B. durch Erwärmen der beiden Reaktionspartner in wässrigem neutralen bis schwach alkalischen Milieu erhalten werden, wobei die Reaktionsprodukte RP entweder als Feststoff aus der Lösung ausfallen oder durch Eindampfen der Lösung daraus isoliert werden. Ferner besteht die Möglichkeit, die Reaktionsprodukte gemäß Literatur H. Baumann et al, J. Liebigs Ann. Chem., 1968, 717, 124-136 oder DE-A1-2165913 darzustellen.

Zur Synthese der Reaktionsprodukte RP können Molverhältnisse der Komponente B zu der Verbindung gemäß Formel I von etwa 1:1 bis etwa 2:1 sinnvoll sein.

Die voranstehend genannten Verbindungen mit der Formel I, die Verbindungen der Komponente B, Komponente C sowie die Reaktionsprodukte RP werden jeweils vorzugsweise in einer Menge von 0,03 bis 65 mmol, insbesondere von 1 bis 40 mmol, bezogen auf 100 g des gesamten Färbemittels, verwendet.

Zusätzlich können die erfindungsgemäßen Mittel mindestens eine Entwicklerkomponente und gegebenenfalls mindestens eine Kupplerkomponente als Oxidationsfarbstoffvorprodukte enthalten.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1)

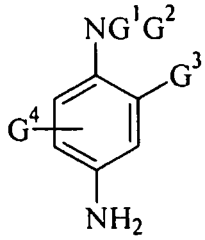

(E1)

wobei

- $G^1$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen 4'-Aminophenylrest oder einen $C_1$- bis $C_4$-Alkylrest, der mit einer stickstoffhaltigen Gruppe, einem Phenyl- oder einem 4'-Aminophenylrest substituiert ist;
- $G^2$ steht für ein Wasserstoffatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest oder einen $C_1$- bis $C_4$-Alkylrest, der mit einer stickstoffhaltigen Gruppe substituiert ist;
- $G^3$ steht für ein Wasserstoffatom, ein Halogenatom, wie ein Chlor-, Brom-, Iod- oder Fluoratom, einen $C_1$- bis $C_4$-Alkylrest, einen $C_1$- bis $C_4$-Monohydroxyalkylrest, einen $C_2$- bis $C_4$-Polyhydroxyalkylrest, einen $C_1$- bis $C_4$-Hydroxyalkoxyrest, einen $C_1$- bis $C_4$-Acetylaminoalkoxyrest, einen $C_1$- bis $C_4$- Mesylaminoalkoxyrest oder einen $C_1$- bis $C_4$-Carbamoylaminoalkoxyrest;
- $G^4$ steht für ein Wasserstoffatom, ein Halogenatom oder einen $C_1$- bis $C_4$-Alkylrest oder
- wenn $G^3$ und $G^4$ in ortho-Stellung zueinander stehen, können sie gemeinsam eine verbrückende $\alpha,\omega$-Alkylendioxogruppe, wie beispielsweise eine Ethylendioxygruppe bilden.

Beispiele für die als Substituenten in den erfindungsgemäßen Verbindungen genannten $C_1$- bis $C_4$-Alkylreste sind die Gruppen Methyl, Ethyl, Propyl, Isopropyl und Butyl. Ethyl und Methyl sind bevorzugte Alkylreste. Erfindungsgemäß bevorzugte $C_1$- bis $C_4$-Alkoxyreste sind beispielsweise eine Methoxy- oder eine Ethoxygruppe. Weiterhin können als bevorzugte Beispiele für eine $C_1$- bis $C_4$-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte $C_2$- bis $C_4$-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, $C_1$- bis $C_4$-Monoalkylaminogruppen, $C_1$- bis $C_4$-Dialkylaminogruppen, $C_1$- bis $C_4$-Trialkylammoniumgruppen, $C_1$- bis $C_4$-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N, N-Dipropyl-p-phenylendiamin, 4-Amino-3-methyl-(N,N-diethyl)-anilin, N,N-Bis-(β-hydroxyethyl)-p-phenylendiamin, 4-N, N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl] amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze:

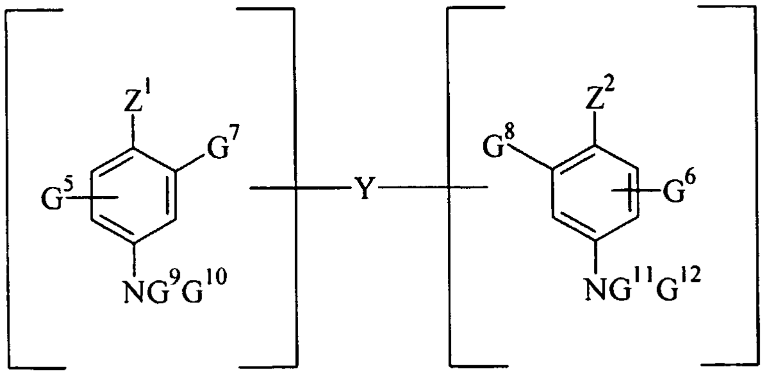

(E2)

wobei:

- $Z^1$ und $Z^2$ stehen unabhängig voneinander für einen Hydroxyl- oder $NH_2$-Rest, der gegebenenfalls durch einen $C_1$- bis $C_4$-Alkylrest, durch einen $C_1$- bis $C_4$-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/ oder einem oder mehreren Heteroatomen wie Sauerstoff-, Schwefel- oder Stickstoffatomen unterbrochen oder beendet sein kann und eventuell durch einen oder mehrere Hydroxyl- oder $C_1$- bis $C_8$-Alkoxyreste substituiert sein kann, oder eine direkte Bindung,
- $G^5$ und $G^6$ stehen unabhängig voneinander für ein Wasserstoff- oder Halogenatom, einen $C_1$- bis $C_4$-Alkylrest, einen $C_1$- bis $C_4$-Monohydroxyalkylrest, einen $C_2$- bis $C_4$- Polyhydroxyalkylrest, einen $C_1$- bis $C_4$-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder einen $C_1$- bis $C_4$-Alkylrest,

mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3)

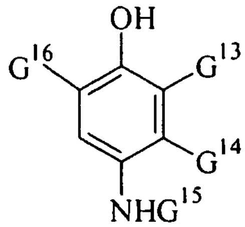

(E3)

wobei:

- $G^{13}$ steht für ein Wasserstoffatom, ein Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$-bis $C_4$)-Alkoxy-($C_1$- bis $C_4$)-alkylrest, einen $C_1$- bis $C_4$-Aminoalkylrest, einen Hydroxy-($C_1$-bis $C_4$)-alkylaminorest, einen $C_1$- bis $C_4$-Hydroxyalkoxyrest, einen $C_1$- bis $C_4$-Hydroxyalkyl-($C_1$-bis $C_4$)-aminoalkylrest oder einen (Di-$C_1$- bis $C_4$-Alkylamino)-($C_1$- bis $C_4$)-alkylrest, und
- $G^{14}$ steht für ein Wasserstoff- oder Halogenatom, einen $C_1$- bis $C_4$-Alkylrest, einen $C_1$- bis $C_4$-Monohydroxyalkylrest, einen $C_2$- bis $C_4$-Polyhydroxyalkylrest, einen ($C_1$- bis $C_4$)-Alkoxy-($C_1$- bis $C_4$)-alkylrest, einen $C_1$- bis $C_4$-Aminoalkylrest oder einen $C_1$- bis $C_4$-Cyanoalkylrest,
- $G^{15}$ steht für Wasserstoff, einen $C_1$- bis $C_4$-Alkylrest, einen $C_1$- bis $C_4$-Monohydroxyalkylrest, einen $C_2$- bis $C_4$-Polyhydroxyalkylrest, einen Phenylrest oder einen Benzylrest, und
- $G^{16}$ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB

1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht:

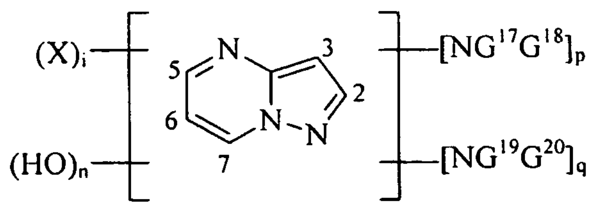

**(E4)**

wobei:

- $G^{17}$, $G^{18}$, $G^{19}$ und $G^{20}$ unabhängig voneinander stehen für ein Wasserstoffatom, einen $C_1$- bis $C_4$-Alkylrest, einen Aryl-Rest, einen $C_1$- bis $C_4$-Hydroxyalkylrest, einen $C_2$-bis $C_4$-Polyhydroxyalkylrest einen ($C_1$- bis $C_4$)-Alkoxy-($C_1$- bis $C_4$)-alkylrest, einen $C_1$- bis $C_4$-Aminoalkylrest, der gegebenenfalls durch ein Acetyl-Ureid- oder einen Sulfonyl-Rest geschützt sein kann, einen ($C_1$- bis $C_4$)-Alkylamino-($C_1$- bis $C_4$)-alkylrest, einen Di-[($C_1$-bis $C_4$)-alkyl]-($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$-bis $C_4$-Hydroxyalkyl- oder einen Di-($C_1$- bis $C_4$)-[Hydroxyalkyl]-($C_1$- bis $C_4$)-aminoalkylrest,
- die X-Reste stehen unabhängig voneinander für ein Wasserstoffatom, einen $C_1$-bis $C_4$-Alkylrest, einen Aryl-Rest, einen $C_1$- bis $C_4$-Hydroxyalkylrest, einen $C_2$- bis $C_4$-Polyhydroxyalkylrest, einen $C_1$- bis $C_4$-Aminoalkylrest, einen ($C_1$- bis $C_4$)-Alkylamino-($C_1$-bis $C_4$)-alkylrest, einen Di-[($C_1$- bis $C_4$)alkyl]- ($C_1$- bis $C_4$)-aminoalkylrest, wobei die Dialkyl-Reste gegebenenfalls einen Kohlenstoffzyklus oder einen Heterozyklus mit 5 oder 6 Kettengliedern bilden, einen $C_1$- bis $C_4$-Hydroxyalkyl- oder einen Di-($C_1$- bis $C_4$-hydroxyalkyl)-aminoalkylrest, einen Aminorest, einen $C_1$- bis $C_4$-Alkyl- oder Di-($C_1$- bis $C_4$-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,

mit der Maßgabe, dass

- die Summe aus p + q ungleich 0 ist,
- wenn p + q gleich 2 ist, n den Wert 0 hat, und die Gruppen $NG^{17}G^{18}$ und $NG^{19}G^{20}$ belegen die Positionen (2,3); (5,6); (6,7); (3,5) oder (3,7);
- wenn p + q gleich 1 ist, n den Wert 1 hat, und die Gruppen $NG^{17}G^{18}$ (oder $NG^{19}G^{20}$) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

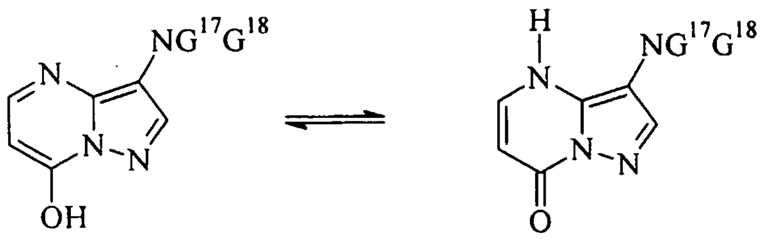

Unter den Pyrazol-[1,5-a]-pyrimidinen der obenstehenden Formel (E4) kann man insbesondere nennen:

- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;

sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind

- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino) ethanol, 2-({3-[(2-Hydroxyethyl)amino]-2-methoxy-5-methylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,

- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol

sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Zusätzlich können im Rahmen einer fünften Ausführungsform als Vorstufen naturanaloger Farbstoffe bevorzugt solche Indole und Indoline in den erfindungsgemäßen Mitteln eingesetzt werden, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel IIIa,

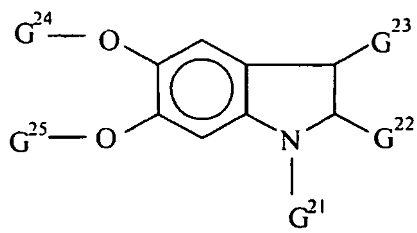

**(IIIa)**

in der unabhängig voneinander

- $G^{21}$ steht für Wasserstoff, eine $C_1$-$C_4$-Alkylgruppe oder eine $C_1$-$C_4$-Hydroxy-alkylgruppe,
- $G^{22}$ steht für Wasserstoff oder eine -COOH-Gruppe, wobei die -COOH-Gruppe auch als Salz mit einem physiologisch verträglichen Kation vorliegen kann,
- $G^{23}$ steht für Wasserstoff oder eine $C_1$-$C_4$-Alkylgruppe,
- $G^{24}$ steht für Wasserstoff, eine $C_1$-$C_4$-Alkylgruppe oder eine Gruppe -CO-$G^{26}$, in der $G^{26}$ steht für eine $C_1$-$C_4$-Alkylgruppe, und
- $G^{25}$ steht für eine der unter $G^{24}$ genannten Gruppen,
  sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel IIIb,

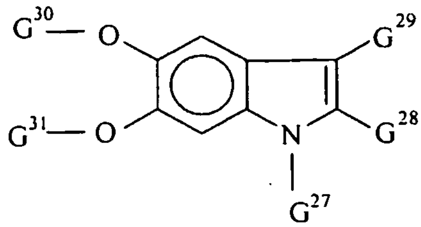

(IIIb)

in der unabhängig voneinander

- $G^{27}$ steht für Wasserstoff, eine $C_1$-$C_4$-Alkylgruppe oder eine $C_1$-$C_4$-Hydroxyalkylgruppe,
- $G^{28}$ steht für Wasserstoff oder eine -COOH-Gruppe, wobei die -COOH-Gruppe auch als Salz mit einem physiologisch verträglichen Kation vorliegen kann,
- $G^{29}$ steht für Wasserstoff oder eine $C_1$-$C_4$-Alkylgruppe,
- $G^{30}$ steht für Wasserstoff, eine $C_1$-$C_4$-Alkylgruppe oder eine Gruppe -CO-$G^{32}$, in der $G^{32}$ steht für eine $C_1$-$C_4$-Alkylgruppe, und
- $G^{31}$ steht für eine der unter $G^{30}$ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Auf die Anwesenheit von Oxidationsmitteln, z. B. $H_2O_2$, kann, insbesondere wenn das erfindungsgemäße Mittel keine Oxidationsfarbstoffvorprodukte enthält, verzichtet werden. Wenn das erfindungsgemäße Mittel luftoxidable Oxidationsfarbstoffvorprodukte oder Indol bzw. Indolinderivate enthält, kann in einem solchen Fall ohne Probleme auf Oxidationsmittel verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist $H_2O_2$. Auch Gemische von mehreren Oxidationsmitteln, wie beispielsweise eine Kombination aus Wasserstoffperoxid und Peroxodisulfaten der Alkali- und Erdalkalimetalle oder aus Iodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze, Metallchelat-Komplexe oder Metalloxide, die einen leichten Wechsel zwischen zwei Oxidationsstufen der Metallionen ermöglichen. Beispiele sind Salze, Chelatkomplexe oder Oxide von Eisen, Ruthenium, Mangan und Kupfer. Weitere mögliche Oxidationskatalysatoren stellen Enzyme dar. Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.

- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,

- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

In einer sechsten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, wie Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl) amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel bevorzugt einen kationischen direktziehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

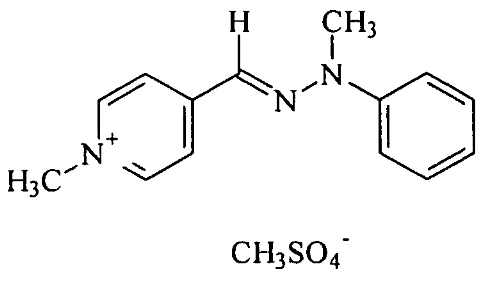

(DZ1) (Basic Yellow 87)

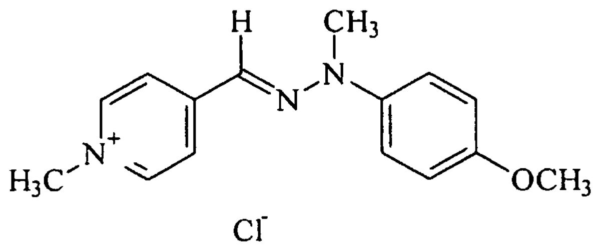

(DZ2)

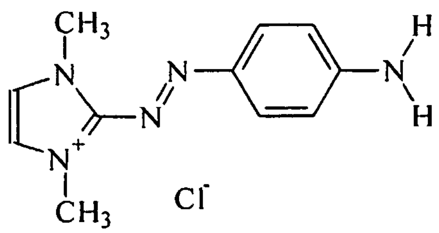

(DZ3) (Basic Orange 31)

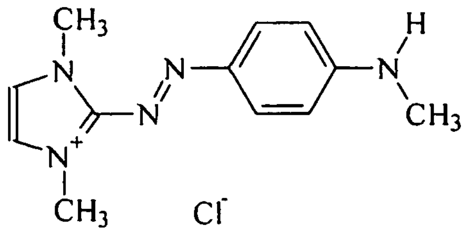

(DZ4)

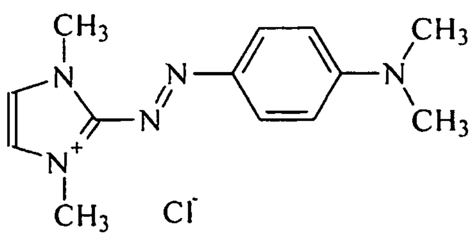

(DZ5) (Basic Red 51)

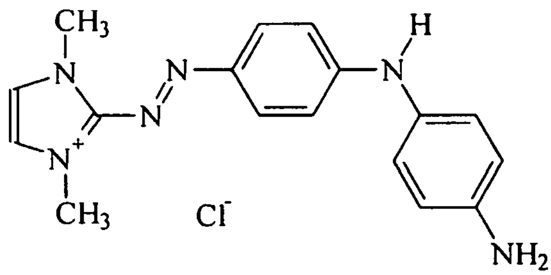

(DZ6)

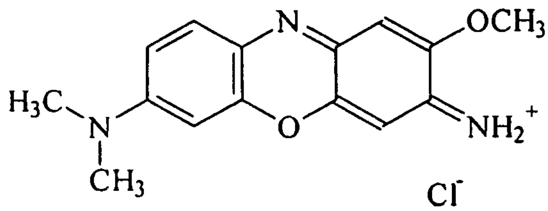

(DZ7)

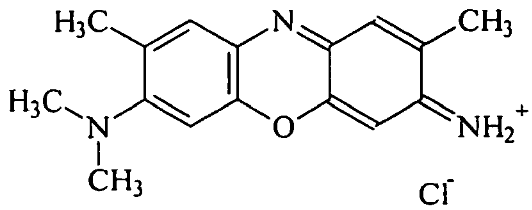

(DZ8)

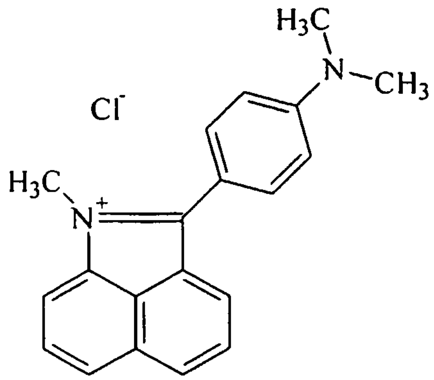

(DZ9)

Die Verbindungen der Formeln (DZ1), (DZ3) und (DZ5) sind ganz besonders bevorzugte kationische direktziehende Farbstoffe der Gruppe (c). Die kationischen direktziehenden Farbstoffe, die unter dem Warenzeichen Arianor® vertrieben werden, sind erfindungsgemäß besonders bevorzugte direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, daß die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,

- lineare Fettsäuren mit 10 bis 22 C-Atomen (Seifen),
- Ethercarbonsäuren der Formel $R-O-(CH_2-CH_2O)_x-CH_2-COOH$, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel $R-O(CH_2-CH_2O)_x-SO_3H$, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten $C_8$-$C_{22}$-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer $C_{8-18}$-Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12-22}$-Fettsäuremono- und -diester von Anlagerungsprodukten von 1 bis 30 Mol Ethylenoxid an Glycerin,
- $C_{8-22}$-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise

- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose

und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,

- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, $N_2O$, Dimethylether, $CO_2$ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein zweiter Gegenstand der vorliegenden Erfindung betrifft die Verwendung von mindestens einer Verbindung gemäß Formel I und/oder deren Enaminform,

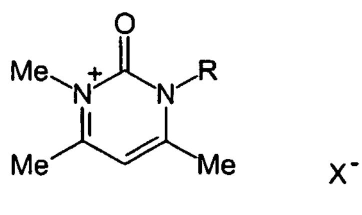

(I)

wobei

- R für eine Allylgruppe, eine Hydroxy-($C_2$- bis $C_6$)-alkylgruppe oder eine gegebenenfalls substituierte Benzylgruppe steht,
- $X^-$ ein physiologisch verträgliches Anion bedeutet,

zusammen mit mindestens einem Aldehyd als Komponente B ausgewählt aus der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd und 4-Dimethylamino-2-methoxybenzaldehyd,
als färbende Komponente in Haarfärbemitteln.

In einer bevorzugten Ausführungsform verwendet man diejenigen Verbindungen gemäß Formel I als färbende-Komponente in Haarfärbemitteln, welche aus den im ersten Erfindungsgegenstand benannten bevorzugten und besonders bevorzugten Vertretern ausgewählt werden.

Darüber hinaus kann es bevorzugt sein, mindestens ein Reaktionsprodukt RP aus einer Verbindung gemäß Formel I und einem Vertreter der Komponente B als färbende Komponenten in Haarfärbemitteln zu verwenden.

Ein dritter Gegenstand der vorliegenden Erfindung betrifft ein Verfahren zum Färben von keratinhaltigen Fasern, insbesondere menschlichen Haaren, worin ein Färbemittel, enthaltend mindestens eine Verbindung gemäß Formel I und/oder deren Enaminform,

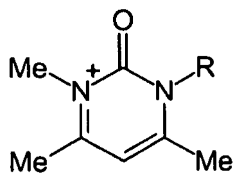

$X^-$ (I)

wobei

- R für eine Allylgruppe, eine Hydroxy-($C_2$- bis $C_6$)-alkylgruppe oder eine gegebenenfalls substituierte Benzylgruppe steht,
- $X^-$ ein physiologisch verträgliches Anion bedeutet,

zusammen mit mindestens einem Aldehyd als Komponente B ausgewählt aus der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd und 4-Dimethylamino-2-methoxybenzaldehyd,
sowie übliche kosmetische Inhaltsstoffe, auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird. Während der Einwirkzeit des Mittels auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partie mit einer Haube abgedeckt.

Dabei können die Verbindungen gemäß Formel I und die Verbindungen der Komponente B, insbesondere deren vorstehend benannte bevorzugte und besonders bevorzugte Vertreter, als farbgebende Komponenten entweder gleichzeitig auf das Haar aufgebracht werden oder aber auch nacheinander, d. h. in einem mehrstufigen Verfahren, wobei es unerheblich ist, welche der Komponenten zuerst aufgetragen wird. Die fakultativ enthaltenen Ammonium- oder Metallsalze können dabei den Verbindungen mit der Formel I oder den Verbindungen der Komponente B zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Vor der Anwendung des erfindungsgemäßen Mittels in dem erfindungsgemäßen Verfahren kann es wünschenswert sein, die zu färbende keratinhaltige Faser einer Vorbehandlung zu unterziehen. Die zeitliche Abfolge des dazu erforderlichen Vorbehandlungsschritts und der Anwendung des erfindungsgemäßen Mittels muß nicht unmittelbar nacheinander sein, sondern es kann zwischen dem Vorbehandlungsschritt und der Anwendung des erfindungsgemäßen Mittels ein Zeitraum von bis maximal zwei Wochen liegen. Dazu eignen sich mehrere Vorbehandlungsmethoden. Bevorzugt wird die Faser

V1 vor der Anwendung des erfindungsgemäßen Mittels einer Blondierung oder
V2 vor der Anwendung des erfindungsgemäßen Mittels einer oxidativen Färbung

unterzogen.

Im Rahmen der Vorbehandlung V1 wird die keratinhaltige Faser mit einem Blondiermittel behandelt. Das Blondiermittel enthält neben einem Oxidationsmittel, wie üblicherweise Wasserstoffperoxid, bevorzugt mindestens ein als Oxidations- und Bleichverstärker wirksames anorganisches Persalz, wie z.B. ein Peroxodisulfat von Natrium, Kalium oder Ammonium. Färbungen gemäß des erfindungsgemäßen Verfahrens erhalten durch die Vorbehandlung V1 eine besondere Brillanz und Farbtiefe.

Im Rahmen der Vorbehandlung V2 wird ein Mittel enthaltend vorgenannte Oxidationsfarbstoffvorprodukte als Entwickler- und gegebenenfalls Kupplerkomponenten sowie gegebenenfalls vorgenannte Derivate des Indols bzw. Indolins auf die Faser aufgetragen und nach einer Einwirkzeit gegebenenfalls unter Zusatz von vorgenannten geeigneten Oxidationsmitteln auf dem Haar für 5-45 Minuten auf der Keratinfaser belassen. Danach wird das Haar gespült. Durch die anschließende Anwendung des erfindungsgemäßen Mittels kann vorhandenen Oxidationsfärbungen einen neue Farbnuance verliehen werden. Wählt man die Farbnuance des erfindungsgemäßen Mittels in der gleichen Farbnuance der oxidativen Färbung aus, so kann die Färbung vorhandener Oxidationsfärbungen nach dem erfindungsgemäßen Verfahren aufgefrischt werden. Es zeigt sich, daß die Farbauffrischung oder Nuancierung gemäß des erfindungsgemäßen Verfahrens einer Farbauffrischung bzw. Nuancierung allein mit herkömmlichen direktziehenden Farbstoffen in der Farbbrillanz und Farbtiefe überlegen ist.

Enthält das Haarfärbemittel neben den Verbindungen gemäß Formel I und den Verbindungen der Komponente B zusätzlich als Oxidationsmittel Wasserstoffperoxid oder ein wasserstoffperoxidhaltiges Oxidationsmittelgemisch, so liegt der pH-Wert des wasserstoffperoxidhaltigen Haarfärbemittels vorzugsweise in einem pH-Bereich von pH 7 bis pH 11, besonders bevorzugt pH 8 bis pH 10. Das Oxidationsmittel kann unmittelbar vor der Anwendung mit dem Haarfärbemittel gemischt und die Mischung auf das Haar aufgebracht werden. Werden die Verbindungen der Formel I und die Komponente B in einem zweistufigen Verfahren auf das Haar appliziert, ist das Oxidationsmittel in einer der beiden Verfahrensstufen zusammen mit der entsprechenden farbgebenden Komponente anzuwenden. Zu diesem Zweck kann es bevorzugt sein, das Oxidationsmittel mit einer der farbgebenden Komponenten in einem Container zu konfektionieren.

Die Verbindungen gemäß Formel I und die Verbindungen der Komponente B können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

Ein vierter Gegenstand der Erfindung ist die Verwendung von mindestens einer Verbindung gemäß Formel I und/oder deren Enaminform,

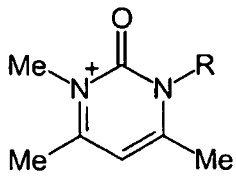

$X^-$ (I)

wobei

- R für eine Allylgruppe, eine Hydroxy-($C_2$- bis $C_6$)-alkylgruppe oder eine gegebenenfalls substituierte Benzylgruppe steht,
- $X^-$ ein physiologisch verträgliches Anion bedeutet,

zusammen mit mindestens einem Aldehyd als Komponente B ausgewählt aus der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd und 4-Dimethylamino-2-methoxybenzaldehyd,
zur Nuancierung von Oxidationsfärbungen von keratinhaltigen Fasern, insbesondere menschlichen Haaren. Bei der Verwendung ist es unerheblich, ob die Nuancierung gleichzeitig während der oxidativen Färbung erfolgt, oder die oxidative Färbung zeitlich vor der Nuancierung liegt.

Ein fünfter Gegenstand der Erfindung ist die Verwendung von mindestens einer Verbindung gemäß Formel I und/oder deren Enaminform,

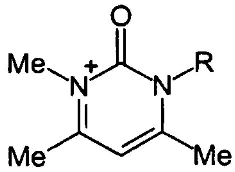

X- (I)

wobei

- R für eine Allylgruppe, eine Hydroxy-($C_2$- bis $C_6$)-alkylgruppe oder eine gegebenenfalls substituierte Benzylgruppe steht,
- $X^-$ ein physiologisch verträgliches Anion bedeutet,

zusammen mit mindestens einem Aldehyd als Komponente B ausgewählt aus der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd und 4-Dimethylamino-2-methoxybenzaldehyd
zur Farbauffrischung von mit oxidativen Färbemitteln gefärbten keratinhaltigen Fasern.

Die Färbungen keratinhaltiger Fasern sind bekanntermaßen Umwelteinflüssen, wie beispielsweise Licht, Reibung oder Waschungen, ausgesetzt und können dadurch an Brillanz und Farbtiefe verlieren. Schlimmstenfalls stellt sich gegebenenfalls eine Nuancenverschiebung der Färbung ein. Solche gealterten Färbungen keratinhaltiger Fasern können, wenn der Anwender es wünscht, durch eine Farbauffrischung wieder annähernd in den farblichen Zustand versetzt werden, wie er sich unmittelbar nach der ursprünglichen Färbung präsentierte. Es ist erfindungsgemäß, für eine solche Farbauffrischung eine Kombination aus mindestens einer Verbindung mit der Formel I und mindestens einer Verbindung der Komponente B zu verwenden.

Ein sechster Erfindungsgegenstand sind Verbindungen gemäß Formel I,

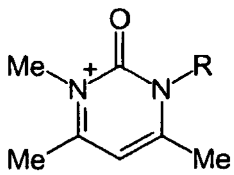

X- (I)

wobei

- R für eine Allylgruppe, eine Hydroxy-($C_2$- bis $C_6$)-alkylgruppe oder eine gegebenenfalls substituierte Benzylgruppe steht und
- $X^-$ ein physiologisch verträgliches Anion bedeutet.

Bevorzugte und besonders bevorzugte Vertreter für R und $X^-$ gemäß Formel (I) sind die Vertreter des ersten Erfindungsgegenstandes.

Besonders bevorzugt sind die Verbindungen der Formeln (II-1) bis (II-12)

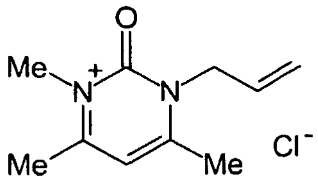

(II-1)

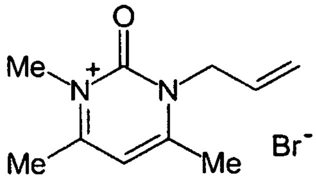

(II-2)

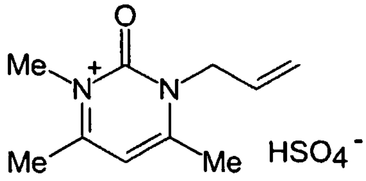

(II-3)

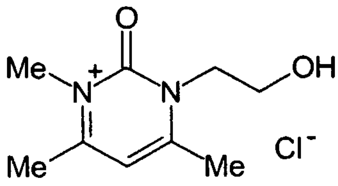

(II-4)

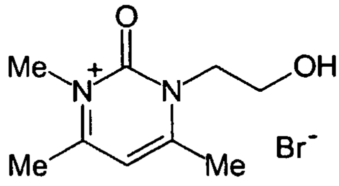

(II-5)

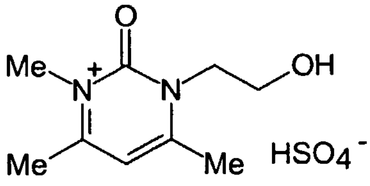

(II-6)

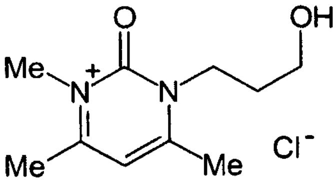

(II-7)

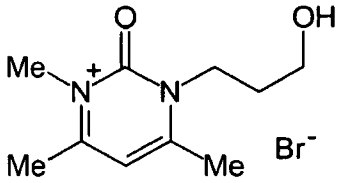

(II-8)

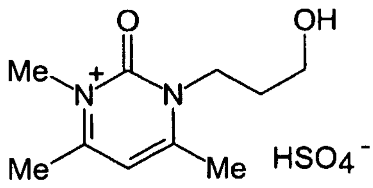

(II-9)

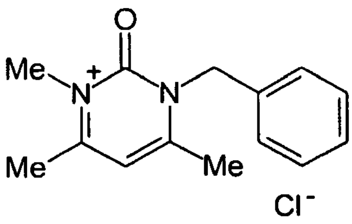

(II-10)

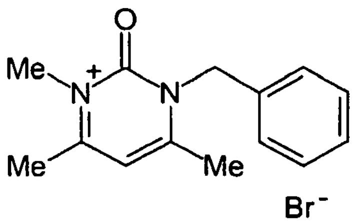

(II-11)

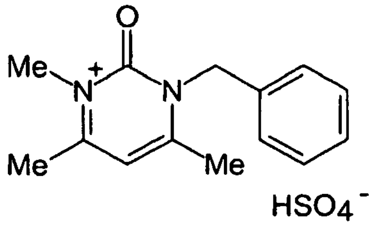

(II-12)

**Beispiele**

**1.0 Synthese der 1,2-Dihydro-3,4,6-trimethyl-2-oxo-pyrimidinium-Derivate**

Synthesebeispiel 1:

1.1.1 Darstellung von 1,4,6-Trimethylpyrimidin-2(1H)-on

Es wurden 20,0 g (0,262 mol) N-Methylharnstoff und 29,4 g (0,291 mol) Acetylaceton zusammen in 100 ml absolutem Ethanol gelöst. Anschließend wurden 97 g konzentrierte Schwefelsäure zugegeben, wobei sich die Reaktionsmischung erwärmte. Anschließend wurde für eine Stunde bei Raumtemperatur nachgerührt. Schon nach kurzer Zeit fiel das Hydrogensulfatsalz des Produktes in Form eines hellen, kristallinen Feststoffes aus, welcher abfiltriert wurde. Dieses Salz wurde in einer geringen Menge Wasser gelöst und die Lösung mit zehnprozentiger Natronlauge neutral gestellt. Anschließend wurde mit Chloroform ausgeschüttelt und das Lösungsmittel mit Magnesiumsulfat getrocknet. Nach Entfernen des Lösungsmittels am Rotationsverdampfer fiel das gewünschte Produkt in Form von blass rosafarbenen Kristallen an.
Smp.: 58 - 60 °C (Lit.: 63 °C; William J. Hale; J. Am. Chem. Soc. 1914, 36, 104 - 115) Ausbeute: 23,6 g (65,2 %)
$^1$H-NMR (400 MHz, DMSO-$d_6$): δ [ppm] = 2,19 (s, 3H); 2,30 (s, 3H); 3,38 (s, 3H); 6,24 (s, 1 H)

1.1.2 Darstellung von 1-Allyl-1,2-dihydro-3,4,6-trimethyl-2-oxopyrimidiniumbromid

Eine Mischung aus 8,0 g (0,058 mol) 1,4,6-Trimethylpyrimidin-2(1H)-on und 14,0 g (0,116 mol) Allylbromid wurden zusammen unter Inertgasatmosphäre für 16 Stunden in Acetonitril unter Rückfluß erhitzt. Nach dem Abkühlen fiel ein violetter Feststoff aus, welcher abgesaugt wurde. Das Produkt wurde durch Umkristallisation aus Chloroform /Diethylether aufgereinigt.
Smp.: 187 - 191 °C
Ausbeute: 11,7 g (77,8 %)
$^1$H-NMR (400 MHz, DMSO-$d_6$): δ [ppm] = 2,63 (s, 3H); 2,69 (s, 3H); 3,67 (s, 3H); 4,75 (d, 2H); 5,72 - 5,80 (2 x dd, 2H); 5,85 - 6,01 (m, 1 H); 7,09 (s, 1 H)

Synthesebeispiel 2:

1.2.1 Darstellung analog der ersten Stufe in Synthesebeispiel 1 unter 1.1.1

1.2.2 Darstellung von 1,2-Dihydro-1-(3-hydroxypropyl)-3,4,6-trimethyl-2-oxopyrimidiniumbromid

Eine Mischung aus 10,0 g (0,072 mol) 1,4,6-Trimethytpyrimidin-2(1H)-on und 20,6 g (0,144 mol) 3-Brompropanol wurde unter Inertgasatmosphäre für 16 Stunden in Acetonitril unter Rückfluß erhitzt. Nach dem Abkühlen fiel ein hellrosafarbener Feststoff aus, welcher abgesaugt wurde (Edukt). Das Filtrat wurde mit Chloroform versetzt, anschließend wurde dieselbe Menge Diethylether zugegeben. Das Produkt schied sich in Form eines Öls ab.
Ausbeute: 15,4 g (77,4 %)
$^1$H-NMR (400 MHz, DMSO-$d_6$): δ [ppm] = 1,72 - 1,86 (m, 2H); 2,53 (s, 3H); 2,73 (s, 3H); 3,47 (t, 2H); 3,65 (s, 3H); 4,18 (t, 2H); 7,11 (s, 1 H)

Synthesebeispiel 3:

3.1.1 Darstellung von 4,6-Dimethyl-1-(2-hydroxyethyl)-pyrimidin-2(1H)on

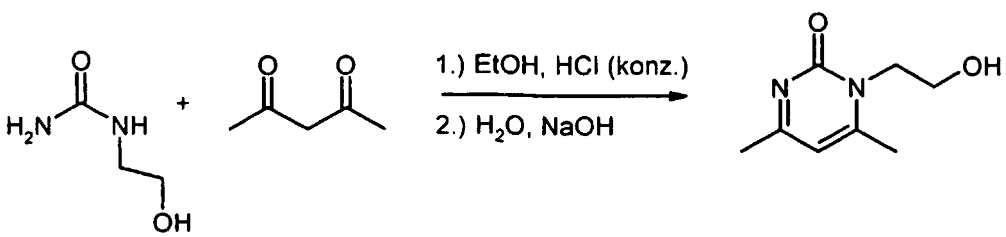


Zu einer Mischung aus 10,0 g (0,091 mol) 2-(Hydroxyethyl)harnstoff und 36,9 g (0,365 mol) Acetylaceton in 140 ml Ethanol wurden 13 g konzentrierte Salzsäure zugetropft. Nach Beendigung des Zutropfens wurde die Reaktionsmischung für 9 Stunden auf 70 °C erhitzt. Nach ca. 1,5 Stunden wurde die ehemals klare Reaktionslösung trüb, und das gewünschte Produkt begann in Form seines Hydrochloridsalzes auszufallen. Nach dem Abkühlen der Reaktionsmischung wurde der Feststoff abfiltriert und in Wasser gelöst. Durch Zugabe von verdünnter Natronlauge wurde ein pH-Wert von 6 - 7 eingestellt, das Wasser unter vermindertem Druck am Rotationsverdampfer entfernt und der Rückstand in Acetonitril aufgenommen. Es wurde vom verbleibenden Feststoff (NaCl) abfiltriert und die organische Phase erneut am Rotationsverdampfer vom Lösungsmittel befreit. Das Produkt fiel in Form eines weißen Pulvers an.

Smp.: 138 - 140 °C (Lit.: 139 - 141 °C; V. S. Reznik et. al. ; Pharmaceutical Chemistry Journal (Translation of Khimiko-Farmatsevticheskü Zhurnal), 2001, 35(12), 672 - 676)
Ausbeute: 10,7 g (69 %)
$^1$H-NMR (400 MHz, DMSO-$d_6$): δ [ppm] = 2,21 (s, 3H); 2,43 (s, 3H); 3,62 (t, 2H); 3,97 (t, 2H); 6,36 (s, 1H)

3.1.2 Darstellung von 1,2-Dihydro-1-(2-hydroxyethyl)-3,4,6-trimethyl-2-oxopyrimidinium-p-toluolsulfonat

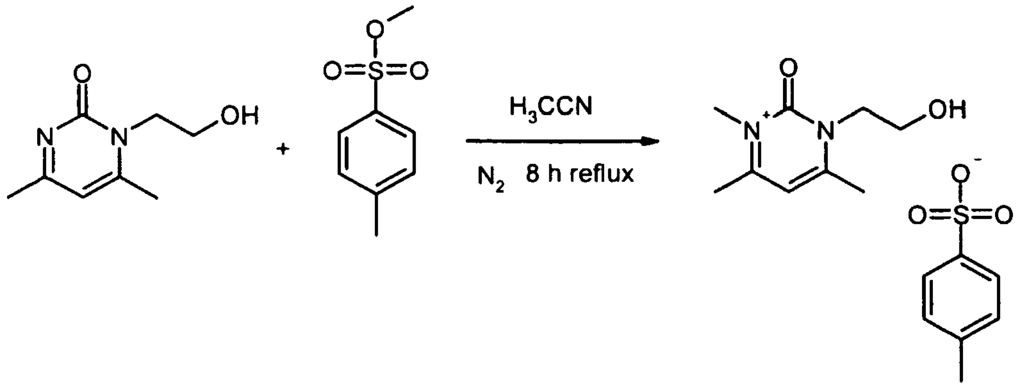


Unter Schutzgasatmosphäre wurde eine Mischung aus 9,2 g (0,055 mol) 4,6-Dimethyl-1-(2-hydroxyethyl)-pyrimidin-2(1H)-on und 13,1 g (0,068 mol) p-Toluolsulfonsäuremethylester in 700 ml Acetonitril für 12 Stunden unter Rückfluß erhitzt. Nach dem Abkühlen wurde ca. 2/3 des Lösungsmittels am Rotationsverdampfer entfernt, der verbleibende Rückstand wurde mit derselben Menge Diethylether vermischt und auf 0 °C gekühlt. In der Kälte schied sich zunächst ein Öl ab, welches nach einiger Zeit auskristallisierte. Das kristalline Produkt wurde abfiltriert.
Smp.. 110 - 115 °C
Ausbeute: 17,1 g (88 %)
$^1$H-NMR (400 MHz, DMSO-$d_6$): δ [ppm] = 2,26 (s, 3H); 2,62 (s, 3H); 2,74 (s, 3H); 3,67 (s, 3H); 3,77 (t, 2H); 4,19 (t, 3H); 7,03 (s, 1H); 7,12 (d, 2H) 7,47 (d, 2H)

**2.0 Herstellung der Färbemittel**

| Wässrige Gelformulierung für Komponente A | Gel 1: |
|---|---|
| CH-acide Verbindung (Komponente A) | 10 mmol |
| Natrosol HR 250 | 2 g |
| Wasser, vollentsalzt | ad 100 g |

Die CH-acide Verbindung (Komponente A) wird zunächst unter Rühren in wenig Wasser gelöst, dann wird mit Wasser auf 98 g aufgefüllt. Unter Rühren wird das Natrosol zugegeben und gewartet, bis es zur gewünschten Verdickung kommt.

| Wässrige Gelformulierung für Komponente B | Gel 2: |
|---|---|
| Carbonylverbindung (Komponente B) | 10 mmol |
| Natrosol HR 250 | 2 g |
| NaOH (50%ige wässrige Lösung) | evtl. einige Tropfen |
| Wasser, vollentsalzt | ad 100 g |

Die Carbonylverbindung (Komponente B) wird in wenig Wasser gelöst bzw. suspendiert. Zur Erhöhung der

Löslichkeit wird bei Bedarf mit einigen Tropfen 50%iger Natronlauge alkalisiert. Anschließend wird mit Wasser auf 98 g aufgefüllt und bis zur vollständigen Lösung der Carbonylverbindung gerührt (teilweise unter gelindem Erwärmen auf ca. 40 °C). Anschließend wird unter Rühren das Natrosol hinzugegeben und der Quellvorgang abgewartet.

**3.0 Ausfärbungen**

Zur Bereitstellung von Egalisiersträhnen wurden Humanhaarsträhnen der Fa. Kerling (0,5g Kerling, Naturweiß) mittig abgebunden und eine Hälfte gebleicht (oberer Strähnenteil). Die andere Hälfte wurde zweimal gebleicht und zwei herkömmlichen Dauerwellbehandlungen mit dem Handelsprodukt Poly Lock-Normale Dauerwelle unterzogen (unterer Strähnenteil). Im Rahmen einer Dauerwellbehandlung wurden die Fasern jeweils in einem ersten Schritt für 30 Minuten bei Raumtemperatur der Reduktionslösung (enthaltend 7,9 Gew.-% Thioglykolsäure) ausgesetzt, mit reinem Wasser gespült und anschließend bei Raumtemperatur für 10 Minuten fixiert (Oxidationslösung, enthaltend 2,6 Gew.-% Wasserstoffperoxid). Nach der oxidativen Behandlung wurden die Fasern jeweils erneut gespült und getrocknet.

Es wurden wässrige Gelformulierungen aus Punkt 2.0 (Gel 1 und Gel 2) mit den Farbstoffvorprodukt Kombinationen der Tabelle 1 hergestellt. Die Gele wurden im Gewichtsverhältnis 1 : 1 vermischt, dann wurde der pH-Wert mit Ammoniak bzw. Weinsäure auf einen Wert von 9 eingestellt. Es wurden die Farbstoffvorprodukte in den Kombinationen verwendet, die in Tabelle 1 aufgeführt sind.

Das erhaltene gebrauchsfertige Haarfärbemittel wurde auf eine Egalisiersträhne aufgebracht (Flotten-Gewichtsverhältnis: Gelmischung zu Haare = 2 zu 1) und mit einer Applicette gleichmäßig verteilt. Nach einer Einwirkzeit von 30 Minuten bei 32 °C wurde die Strähne mit lauwarmem Wasser ausgespült und danach im warmen Luftstrom (30°C bis 40°C) getrocknet. Es wurden mit den erfindungemäßen Farbstoffvorprodukten intensivere und brillantere Färbungen erhalten. Die Färbungen sind der Tabelle 1 zu entnehmen.

Verbindungen der Komponente A (Tabelle 1):

A1 1,2-Dihydro-1,3,4-trimethyl-2-oxo-pyrimidinium-chlorid (nicht erfindungsgemäß)
A2 1,2-Dihydro-1,3,4,6-tetramethyl-2-oxo-pyrimidinium-hydrogensulfat (nicht erfindungsgemäß)
A3 1-Allyl-1,2-dihydro-3,4,6-trimethyl-2-oxo-pyrimidinium-bromid (erfindungsgemäß)
A4 1,2-Dihydro-1-(2-hydroxyethyl)-3,4,6-trimethyl-2-oxopyrimidinium-p-toluolsulfonat (erfindungsgemäß)

Verbindungen der Komponente B (Tabelle 1):

B1 4-Hydroxy-3-methoxybenzaldehyd (Vanillin)
B2 3,5-Dimethoxy-4-hydroxy-benzaldehyd
B3 4-Hydroxy-1-naphthaldehyd
B4 4-Hydroxy-2-methoxybenzaldehyd
B5 3,4,5-Trihydroxybenzaldehyd
B6 3,4-Dihydroxy-5-methoxybenzaldehyd
B7 2,4-Dimethoxybenzaldehyd (nicht erfindungsgemäß)
B8 4-Hydroxybenzaldehyd (nicht erfindungsgemäß)

Tabelle 1:

| Komponente A (Gel 1) | Komponente B (Gel 2) | Farbton | erfindungsgemäß |
|---|---|---|---|
| A1 | B1 | violett | nein |
| A1 | B7 | gelb | nein |
| A1 | B8 | orangerot | nein |
| A2 | B1 | violett | nein |
| A2 | B2 | dunkelblau | nein |

(fortgesetzt)

| Komponente A (Gel 1) | Komponente B (Gel 2) | Farbton | erfindungsgemäß |
|---|---|---|---|
| A2 | B4 | rot | nein |
| A3 | B1 | leuchtend violett | ja |
| A3 | B2 | intensiv dunkelblau | ja |
| A3 | B3 | intensiv dunkelblau | ja |
| A3 | B4 | leuchtend rot | ja |
| A3 | B5 | intensiv dunkelblau | ja |
| A3 | B6 | intensiv dunkelblau | ja |
| A4 | B1 | leuchtend violett | ja |
| A4 | B2 | intensiv dunkelblau | ja |
| A4 | B3 | leuchtend violett | ja |
| A4 | B4 | leuchtend rot | ja |

4.0 Bestimmung der Waschechtheit

Nach dem Färbevorgang aus 3.0 wurden die Egalisiersträhnen farbmetrisch mit einem Farbmessgerät der Firma Datacolor, Typ Spectraflash 450 vermessen. Es erfolgten vier Messungen im oberen Teil der Haarsträhne, sowie vier Messungen im unteren Teil der Strähne. Zur Simulation des Auswaschvorgangs wurden die Haarsträhnen für 15 Minuten in ein Ultraschallbad der Firma Elma (Typ T 790/H, Stufe 5), welches mit einer 1%igen Texapon-NSO-UP Lösung befüllt war, eingebracht. Nach dem Trocknen erfolgte eine erneute farbmetrische Vermessung wie oben beschrieben.

Der für die Beurteilung der Waschechtheit herangezogene dE-Wert je Strähnenteil ergibt sich aus den am jeweiligen Strähnenteil gemessenen L*a*b*-Farbmesswerten wie folgt:

$$dE = [\,(L_i - L_0)^2 + (a_i - a_0)^2 + (b_i - b_0)]^{1/2}$$

$L_0$, $a_0$ und $b_0$ sind hierbei jeweils die Mittelwerte der aus den vier Messungen ermittelten Farbmesswerte vor dem Waschversuch, während $L_i$, $a_i$ und $b_i$ die gemittelten Farbmesswerte nach dem Waschversuch darstellen.

Die Waschechtheit einer Haarfarbe ist umso schlechter, je größer der dE-Wert ist. Die dE-Werte sind in Tabelle 2 zusammengefaßt. Die Färbungen mit den erfindungsgemäßen Kombinationen der Komponenten A und B weisen im Vergleich zu den Kombinationen des Standes der Technik eine bessere Waschechtheit auf.

Verbindungen der Komponente A (Tabelle 2):

| | |
|---|---|
| A1 | 1,2-Dihydro-1,3,4-trimethyl-2-oxo-pyrimidinium-chlorid (nicht erfindungsgemäß) |
| A2 | 1,2-Dihydro-1,3,4,6-tetramethyl-2-oxo-pyrimidinium-hydrogensulfat (nicht erfindungsgemäß) |
| A3 | 1-Allyl-1,2-dihydro-3,4,6-trimethyl-2-oxo-pyrimidinium-bromid |

Verbindungen der Komponente B (Tabelle 2):

| | |
|---|---|
| B1 | 4-Hydroxy-3-methoxybenzaldehyd (Vanillin) |
| B2 | 3,5-Dimethoxy-4-hydroxy-benzaldehyd |
| B3 | 4-Hydroxy-1-naphthaldehyd |
| B4 | 4-Hydroxy-2-methoxybenzaldehyd |

Tabelle 2:

| Komponente A (Gel 1) | Komponente B (Gel 2) | dE oberer Strähenteil | dE unterer Strähnenteil | gemäß Erfindung |
|---|---|---|---|---|
| A1 | B1 | 9,3 | 34,3 | nein |
| A2 | B1 | 3,5 | 4,0 | nein |
| A2 | B2 | 4,8 | 4,1 | nein |
| A2 | B4 | 7,9 | 2,0 | nein |
| A3 | B1 | 1,8 | 1,7 | ja |
| A3 | B2 | 1,6 | 2,5 | ja |
| A3 | B3 | 3,7 | 5,6 | ja |
| A3 | B4 | 1,1 | 1,7 | ja |

5.0 Bestimmung der Lichtechtheit

Die Haarsträhne wurde nach der Färbung gemäß Punkt 3.0 für 120 Stunden der Bestrahlung mit einem Xenon-Brenner nach DIN 54004 (Farbtemperatur 5500-6500 K; Wellenlängenbereiche: UV-Bereich = 300-400 nm; Vis-Bereich = 400-700 nm) ausgesetzt. Nach dieser Behandlung wurde die Strähne visuell unter einer Tageslichtlampe beurteilt. Die Farbintensität wurde mit einem Wert zwischen 1 und 6 benotet. Der Wert 6 bedeutet hierbei eine sehr gute Lichtechtheit, bei einem Wert von 1 ist die Lichtechtheit als sehr schlecht einzustufen. Die Beurteilung der Lichtechtheiten ist in Tabelle 3 zusammengefasst. Die erfindungsgemäßen Färbungen besitzen stark verbesserte Lichtechtheiten.

Tabelle 3:

| Komponente A (Gel 1) | Komponente B (Gel 2) | Lichtechtheit | erfindungsgemäß |
|---|---|---|---|
| A1 | B7 | 1 - 2 | nein |
| A1 | B8 | 1 - 2 | nein |
| A3 | B5 | 5 - 6 | ja |
| A3 | B6 | 6 | ja |

Verbindungen der Komponente A (Tabelle 3):

A1 1,2-Dihydro-1,3,4-trimethyl-2-oxo-pyrimidinium-chlorid (nicht erfindungsgemäß)
A3 1-Allyl-1,2-dihydro-3,4,6-trimethyl-2-oxo-pyrimidinium-bromid

Verbindungen der Komponente B (Tabelle 3):

B5 3,4,5-Trihydroxybenzaldehyd
B6 3,4-Dihydroxy-5-methoxybenzaldehyd
B7 2,4-Dimethoxybenzaldehyd (nicht erfindungsgemäß)
B8 4-Hydroxybenzaldehyd (nicht erfindungsgemäß)

**Patentansprüche**

**1.** Mittel zum Färben von keratinhaltigen Fasern, insbesondere menschlichen Haaren, enthaltend als Komponente A

mindestens eine Verbindung gemäß Formel I und/oder deren Enaminform,

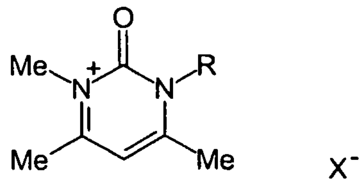

(I)

wobei

- R für eine Allylgruppe, eine Hydroxy-($C_2$- bis $C_6$)-alkylgruppe oder eine gegebenenfalls substituierte Benzylgruppe steht,
- $X^-$ ein physiologisch verträgliches Anion bedeutet

und als Komponente B mindestens einen Aldehyd ausgewählt aus der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd und 4-Dimethylamino-2-methoxybenzaldehyd.

**2.** Mittel nach Anspruch 1, **dadurch gekennzeichnet, daß** R gemäß Formel (I) für eine Allylgruppe, eine 3-Hydroxypropylgruppe, eine 2-Hydroxypropylgruppe, eine 2-Hydroxyethylgruppe oder eine Benzylgruppe steht.

**3.** Mittel nach einem der Ansprüche 1 oder 2, **dadurch gekennzeichnet, daß** $X^-$ steht für Halogenid, Benzolsulfonat, p-Toluolsulfonat, ($C_1$- bis $C_4$)-Alkansulfonat, Trifluormethansulfonat, Perchlorat, 0.5 Sulfat, Hydrogensulfat, Tetrafluoroborat, Hexafluorophosphat oder Tetrachlorozinkat.

**4.** Mittel nach einem der Ansprüche 1 bis 3, **dadurch gekennzeichnet, daß** die Verbindung der Formel (I) aus der Gruppe ausgewählt wird, bestehend aus physiologisch verträglichen Salzen des 1-Allyl-1,2-dihydro-3,4,6-trimethyl-2-oxopyrimidiniums, des 1,2-Dihydro-1-(2-hydroxyethyl)-3,4,6-trimethyl-2-oxopyrimidiniums, des 1,2-Dihydro-1-(3-hydroxypropyl)-3,4,6-trimethyl-2-oxopyrimidiniums und des 1-Benzyl-1,2-dihydro-3,4,6-trimethyl-2-oxo-pyrimidiniums und den Enaminformen der vorgenannten Salze.

**5.** Mittel nach einem der Ansprüche 1 bis 4, **dadurch gekennzeichnet, daß** es zusätzlich mindestens eine Verbindung als Komponente C enthält, ausgewählt aus (a) CH-aciden Verbindungen, welche von Verbindungen der Formel (I) gemäß Anspruch 1 verschiedenen sind, und/oder (b) reaktiven Carbonylverbindungen, welche von den Verbindungen der Komponente B gemäß Anspruch 1 verschieden sind.

**6.** Mittel nach Anspruch 5, **dadurch gekennzeichnet, daß** die CH-aciden Verbindungen der Komponente C ausgewählt werden, aus der Gruppe, bestehend aus mit physiologisch verträglichen Anionen gebildeten Salzen des 1,4-Dimethylchinoliniums, 1-Ethyl-4-methyl-chinoliniums, 1-Ethyl-2-methylchinoliniums, 1,2,3,3-Tetramethyl-3H-indoliums, 2,3-Dimethyl-benzothiazoliums, 2,3-Dimethylnaphtho[1,2-d]thiazoliums, 3-Ethyl-2-methyl-naphtho[1,2-d]thiazoliums, 3-Ethyl-2-methyl-benzoxazoliums, 1,2,3-Trimethylchinoxaliniums, 3-Ethyl-2-methylbenzothiazoliums, 1,2-Dihydro-1,3,4,6-tetramethyl-2-oxo-pyrimidiniums, 1,2-Dihydro-1,3,4-trimethyl-2-oxo-pyrimidiniums, 1,2-Dihydro-4,6-dimethyl-1,3-dipropyl-2-oxo-pyrimidiniums, 1,2-Dihydro-1,3,4,6-tetramethyl-2-thioxo-pyrimidiniums, 1,2-Dihydro-1,3,4,5,6-pentamethyl-2-oxo-pyrimidiniums, 2,5-Dimethyl-3-(2-propenyl)-1,3,4-thiadiazoliums, 3-Ethyl-2,5-dimethyl-1,3,4-thiadiazoliums, 1,2-Dimethylchinoliniums und 1,3,3-Trimethyl-2-methylenindolin (Fischersche Base), Oxindol, 3-Methyl-1-phenyl-pyrazolin-5-on, Indan-1,2-dion, Indan-1,3-dion, Indan-1-on, 2-Amino-4-imino-1,3-thiazolin-hydrochlorid, Benzoylacetonitril, 3-Dicyanmethylenindan-1-on, 2-(2-Furanoyl)acetonitril, 2-(2-Theonyl)acetonitril, 2-(Cyanmethyl)benzimidazol, 2-(Cyanmethyl)-benzothiazol und 2-(2,5-Dimethyl-3-furanoyl)acetonitril.

**7.** Mittel nach einem der Ansprüche 5 oder 6, **dadurch gekennzeichnet, daß** die reaktiven Carbonylverbindungen der Komponente C ausgewählt werden aus der Gruppe bestehend aus Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,5-Diethoxy-4-hydroxy-benzaldehyd, 2,6-Diethoxy-4-hydroxy-benzaldehyd, 3-Hydroxy-4-methoxybenzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-(1-Imidazolyl)-benzaldehyd und Piperonal.

8. Mittel nach einem der Ansprüche 1 bis 7, **dadurch gekennzeichnet, daß** die Verbindungen der Formel I, die Verbindungen der Komponente B und die Verbindungen der Komponente C jeweils in einer Menge von 0,03 bis 65 mmol, insbesondere von 1 bis 40 mmol, bezogen auf 100 g des gesamten Färbemittels, enthalten sind.

9. Mittel nach einem der Ansprüche 1 bis 8, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

10. Mittel nach einem der Ansprüche 1 bis 9, **dadurch gekennzeichnet, daß** es zusätzlich Oxidationsmittel, insbesondere $H_2O_2$, in einer Menge von 0,01 bis 6 Gew.-%, bezogen auf die Anwendungslösung, enthält.

11. Mittel nach einem der Ansprüche 1 bis 10, **dadurch gekennzeichnet, daß** es zusätzlich als Oxidationsfarbstoffvorprodukt mindestens eine Entwicklerkomponente und gegebenenfalls mindestens eine Kupplerkomponente enthält.

12. Mittel nach einem der Ansprüche 1 bis 11, **dadurch gekennzeichnet, daß** es zusätzlich mindestens einen direktziehenden Farbstoff, vorzugsweise in einer Menge von 0,01 bis 20 Gew.-%, bezogen auf das gesamte Färbemittel, enthält.

13. Mittel nach einem der Ansprüche 1 bis 12, **dadurch gekennzeichnet, daß** es zusätzlich anionische, zwitterionische oder nichtionische Tenside enthält.

14. Verwendung von mindestens einer Verbindung gemäß Formel I gemäß Anspruch 1, in Kombination mit mindestens

einer Verbindung der Komponente B gemäß Anspruch 1, als eine färbende Komponente in Haarfärbemitteln.

**15.** Verfahren zum Färben von keratinhaltigen Fasern, insbesondere menschlichen Haaren, worin ein Färbemittel, gemäß einem der Ansprüche 1 bis 13, sowie übliche kosmetische Inhaltsstoffe, auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird.

**16.** Verfahren gemäß Anspruch 15, **dadurch gekennzeichnet, daß** in einem Zweischrittverfahren die Komponente B gemäß Anspruch 1 vor oder nach Applikation der Verbindung gemäß Formel I gemäß Anspruch 1 auf die keratinhaltigen Fasern aufgebracht, die auf dem Haar erhaltene Mischung einige Zeit, üblicherweise ca. 15-30 Minuten, auf der Faser belassen und anschließend wieder ausgespült oder mit einem Shampoo ausgewaschen wird.

**17.** Verfahren nach einem der Ansprüche 15 oder 16, **dadurch gekennzeichnet, daß** die keratinhaltigen Fasern, bevor ein Färbemittel gemäß einem der Ansprüche 1 bis 13 zur Anwendung kommt, im Rahmen einer Vorbehandlung mit einem Blondiermittel blondiert oder mit einem Oxidationsfärbemittel gefärbt wurden.

**18.** Verwendung von mindestens einer Verbindung gemäß Formel I und/oder deren Enaminform,

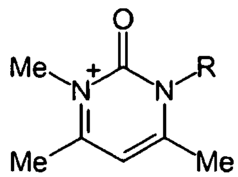

$X^-$ (I)

wobei

- R für eine Allylgruppe, eine Hydroxy-($C_2$- bis $C_6$)-alkylgruppe oder eine gegebenenfalls substituierte Benzylgruppe steht,
- $X^-$ ein physiologisch verträgliches Anion bedeutet

zusammen mit mindestens einem Aldehyd als Komponente B ausgewählt aus der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd und 4-Dimethylamino-2-methoxybenzaldehyd, zur Nuancierung von Oxidationsfärbungen von keratinhaltigen Fasern, insbesondere menschlichen Haaren.

**19.** Verwendung von mindestens einer Verbindung gemäß Formel I und/oder deren Enaminform,

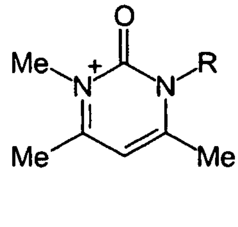

$X^-$ (I)

wobei

- R für eine Allylgruppe, eine Hydroxy-($C_2$- bis $C_6$)-alkylgruppe oder eine gegebenenfalls substituierte Benzylgruppe steht,
- $X^-$ ein physiologisch verträgliches Anion bedeutet

und zusammen mit mindestens einem Aldehyd als Komponente B ausgewählt aus der Gruppe bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd und 4-Dimethylamino-2-methoxybenzaldehyd
zur Farbauffrischung von mit oxidativen Färbemitteln gefärbten keratinhaltigen Fasern.

**20.** Verbindungen gemäß Formel I,

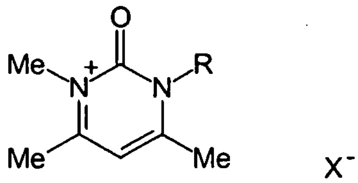

(I)

wobei

- R für eine Allylgruppe, eine Hydroxy-($C_2$- bis $C_6$)-alkylgruppe oder eine gegebenenfalls substituierte Benzylgruppe steht und
- $X^-$ ein physiologisch verträgliches Anion bedeutet.

**Claims**

**1.** A substance for dyeing keratinic fibers, in particular human hair, comprising, as component A, at least one compound according to formula I and/or its enamine form,

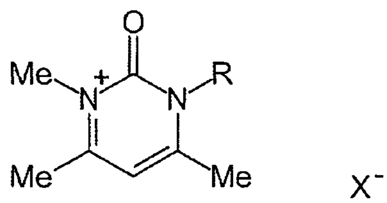

(I)

where

- R is an allyl group, a hydroxy-($C_2$- to $C_6$)-alkyl group or an optionally substituted benzyl group,
- $X^-$ is a physiologically compatible anion

and, as component B, at least one aldehyde chosen from the group consisting of 4-hydroxy-3-methoxybenzaldehyde, 3,5-dimethoxy-4-hydroxybenzaldehyde, 4-hydroxy-1-naphthaldehyde, 4-hydroxy-2-methoxybenzaldehyde, 3,4-dihydroxy-5-methoxybenzaldehyde, 3,4,5-trihydroxybenzaldehyde, 3,5-dibromo-4-hydroxybenzaldehyde, 4-hydroxy-3-nitrobenzaldehyde, 3-bromo-4-hydroxybenzaldehyde, 4-hydroxy-3-methylbenzaldehyde, 3,5-dimethyl-4-hydroxybenzaldehyde, 5-bromo-4-hydroxy-3-methoxybenzaldehyde, 4-diethylamino-2-hydroxybenzaldehyde and 4-dimethylamino-2-methoxybenzaldehyde.

**2.** The substance as claimed in claim 1, **characterized in that** R according to formula (I) is an allyl group, a 3-hydroxypropyl group, a 2-hydroxypropyl group, a 2-hydroxyethyl group or a benzyl group.

**3.** The substance as claimed in one of claims 1 or 2, **characterized in that** $X^-$ is halide, benzenesulfonate, p-toluenesulfonate, ($C_1$- to $C_4$)-alkanesulfonate, trifluoromethanesulfonate, perchlorate, 0.5 sulfate, hydrogensulfate, tetrafluoroborate, hexafluorophosphate or tetrachlorozincate.

4. The substance as claimed in one of claims 1 to 3, **characterized in that** the compound of the formula (I) is chosen from the group consisting of physiologically compatible salts of 1-allyl-1,2-dihydro-3,4,6-trimethyl-2-oxopyrimidinium, of 1,2-dihydro-1-(2-hydroxyethyl)-3,4,6-trimethyl-2-oxopyrimidinium, of 1,2-dihydro-1-(3-hydroxypropyl)-3,4,6-trimethyl-2-oxopyrimidinium and of 1-benzyl-1,2-dihydro-3,4,6-trimethyl-2-oxopyrimidinium and the enamine forms of the abovementioned salts.

5. The substance as claimed in one of claims 1 to 4, **characterized in that** it additionally comprises at least one compound as component C chosen from (a) CH-acidic compounds which are different from compounds of the formula (I) according to claim 1, and/or (b) reactive carbonyl compounds which are different from the compounds of component B according to claim 1.

6. The substance as claimed in claim 5, **characterized in that** the CH-acidic compounds of component C are chosen from the group consisting of salts, formed with physiologically compatible anions, of 1,4-dimethylquinolinium, 1-ethyl-4-methylquinolinium, 1-ethyl-2-methylquinolinium, 1,2,3,3-tetramethyl-3H-indolium, 2,3-dimethylbenzothiazolium, 2,3-dimethylnaphtho[1,2-d]thiazolium, 3-ethyl-2-methylnaphtho[1,2-d]thiazolium, 3-ethyl-2-methylbenzoxazolium, 1,2,3-trimethylquinoxalinium, 3-ethyl-2-methylbenzothiazolium, 1,2-dihydro-1,3,4,6-tetramethyl-2-oxopyrimidinium, 1,2-dihydro-1,3,4-trimethyl-2-oxopyrimidinium, 1,2-dihydro-4,6-dimethyl-1,3-dipropyl-2-oxopyrimidinium, 1,2-dihydro-1,3,4,6-tetramethyl-2-thioxopyrimidinium, 1,2-dihydro-1,3,4,5,6-pentamethyl-2-oxopyrimidinium, 2,5-dimethyl-3-(2-propenyl)-1,3,4-thiadiazolium, 3-ethyl-2,5-dimethyl-1,3,4-thiadiazolium, 1,2-dimethylquinolinium and 1,3,3-trimethyl-2-methyleneindoline (Fischer's base), oxindole, 3-methyl-1-phenylpyrazolin-5-one, indane-1,2-dione, indane-1,3-dione, indan-1-one, 2-amino-4-imino-1,3-thiazoline hydrochloride, benzoylacetonitrile, 3-dicyanomethyleneindan-1-one, 2-(2-furanoyl)acetonitrile, 2-(2-theonyl)acetonitrile, 2-(cyanomethyl)benzimidazole, 2-(cyanomethyl)benzothiazole and 2-(2,5-dimethyl-3-furanoyl)acetonitrile.

7. The substance as claimed in one of claims 5 or 6, **characterized in that** the reactive carbonyl compounds of component C are chosen from the group consisting of coniferylaldehyde, 2-methoxybenzaldehyde, 3-methoxybenzaldehyde, 4-methoxybenzaldehyde, 2-ethoxybenzaldehyde, 3-ethoxybenzaldehyde, 4-ethoxybenzaldehyde, 4-hydroxy-2,3-dimethoxybenzaldehyde, 4-hydroxy-2,5-dimethoxybenzaldehyde, 4-hydroxy-2,6-dimethoxybenzaldehyde, 4-hydroxy-2-methylbenzaldehyde, 4-hydroxy-2,3-dimethylbenzaldehyde, 4-hydroxy-2,5-dimethylbenzaldehyde, 4-hydroxy-2,6-dimethylbenzaldehyde, 3,5-diethoxy-4-hydroxybenzaldehyde, 2,6-diethoxy-4-hydroxybenzaldehyde, 3-hydroxy-4-methoxybenzaldehyde, 2-hydroxy-4-methoxybenzaldehyde, 2-ethoxy-4-hydroxybenzaldehyde, 3-ethoxy-4-hydroxybenzaldehyde, 4-ethoxy-2-hydroxybenzaldehyde, 4-ethoxy-3-hydroxybenzaldehyde, 2,3-dimethoxybenzaldehyde, 2,4-dimethoxybenzaldehyde, 2-5-dimethoxybenzaldehyde, 2,6-dimethoxybenzaldehyde, 3,4-dimethoxybenzaldehyde, 3,5-dimethoxybenzaldehyde, 2,3,4-trimethoxybenzaldehyde, 2,3,5-trimethoxybenzaldehyde, 2,3,6-trimethoxybenzaldehyde, 2,4,6-trimethoxybenzaldehyde, 2,4,5-trimethoxybenzaldehyde, 2,5,6-trimethoxybenzaldehyde, 2-hydroxybenzaldehyde, 3-hydroxybenzaldehyde, 4-hydroxybenzaldehyde, 2,3-dihydroxybenzaldehyde, 2,4-dihydroxybenzaldehyde, 2,4-dihydroxy-3-methylbenzaldehyde, 2,4-dihydroxy-5-methylbenzaldehyde, 2,4-dihydroxy-6-methylbenzaldehyde, 2,4-dihydroxy-3-methoxybenzaldehyde, 2,4-dihydroxy-5-methoxybenzaldehyde, 2,4-dihydroxy-6-methoxybenzaldehyde, 2,5-dihydroxybenzaldehyde, 2,6-dihydroxybenzaldehyde, 3,4-dihydroxybenzaldehyde, 3,4-dihydroxy-2-methylbenzaldehyde, 3,4-dihydroxy-5-methylbenzaldehyde, 3,4-dihydroxy-6-methylbenzaldehyde, 3,4-dihydroxy-2-methoxybenzaldehyde, 3,5-dihydroxybenzaldehyde, 2,3,4-trihydroxybenzaldehyde, 2,3,5-trihydroxybenzaldehyde, 2,3,6-trihydroxybenzaldehyde, 2,4,6-trihydroxybenzaldehyde, 2,4,5-trihydroxybenzaldehyde, 2,5,6-trihydroxybenzaldehyde, 4-dimethylaminobenzaldehyde, 4-diethylaminobenzaldehyde, 4-dimethylamino-2-hydroxybenzaldehyde, 4-pyrrolidinobenzaldehyde, 4-morpholinobenzaldehyde, 2-morpholinobenzaldehyde, 4-piperidinobenzaldehyde, 3,5-dichloro-4-hydroxybenzaldehyde, 4-hydroxy-3,5-diiodobenzaldehyde, 3-chloro-4-hydroxybenzaldehyde, 5-chloro-3,4-dihyroxybenzaldehyde, 5-bromo-3,4-dihydroxybenzaldehydo, 3-chloro-4-hydroxy-5-methoxybenzaldehyde, 4-hydroxy-3-iodo-5-methoxybenzaldehyde, 2-methoxy-1-naphthaldehyde, 4-methoxy-1-naphthaldehyde, 2-hydroxy-1-naphthaldehyde, 2,4-dihydroxy-1-naphthaldehyde, 4-hydroxy-3-methoxy-1-naphthaldehyde, 2-hydroxy-4-methoxy-1-naphthaldehyde, 3-hydroxy-4-methoxy-1-naphthaldehyde, 2,4-dimethoxy-1-naphthaldehyde, 3,4-dimethoxy-1-naphthaldehyde, 4-dimethylamino-1-naphthaldehyde, 2-nitrobenzaldehyde, 3-nitrobenzaldehyde, 4-nitrobenzaldehyde, 4-methyl-3-nitrobenzaldehyde, 3-hydroxy-4-nitrobenzaldehyde, 5-hydroxy-2-nitrobenzaldehyde, 2-hydroxy-5-nitrobenzaldehyde, 2-hydroxy-3-nitrobenzaldehyde, 2-fluoro-3-nitrobenzaldehyde, 3-methoxy-2-nitrobenzaldehyde, 4-chloro-3-nitrobenzaldehyde, 2-chloro-6-nitrobenzaldehyde, 5-chloro-2-nitrobenzaldehyde, 4-chloro-2-nitrobenzaldehyde, 2,4-dinitrobenzaldehyde, 2,6-dinitrobenzaldehyde, 2-hydroxy-3-methoxy-5-nitrobenzaldehyde, 4,5-dimethoxy-2-nitrobenzaldehyde, 6-nitropiperonal, 2-nitropiperonal, 5-nitrovanillin, 2,5-dinitrosalicylaldehyde, 5-bromo-3-nitrosalicylaldehyde, 4-nitro-1-naphthaldehyde, 2-nitrocinnamaldehyde, 3-nitrocinnamaldehyde, 4-nitrocinnamaldehyde, 4-dimethylaminocinnamaldehyde, 2-dimethylaminobenzaldehyde, 2-chloro-4-dimethylaminobenzaldehyde, 4-dimethylamino-2-methylbenzaldehyde,

4-diethylaminocinnamaldehyde, 4-dibutylaminobenzaldehyde, 4-diphenylaminobenzaldehyde, 4-(1-imidazolyl) benzaldehyde and piperonal.

**8.** The substance as claimed in one of claims 1 to 7, **characterized in that** the compounds of the formula I, the compounds of component B and the compounds of component C are in each case present in an amount of from 0.03 to 65 mmol, in particular from 1 to 40 mmol, based on 100 g of the total colorant.

**9.** The substance as claimed in one of claims 1 to 8, **characterized in that** it comprises color enhancers chosen from the group consisting of piperidine, piperidine-2-carboxylic acid, piperidine-3-carboxylic acid, piperdine-4-carboxylic acid, pyridine, 2-hydroxypyridine, 3-hydroxypyridine, 4-hydroxypyridine, imidazole, 1-methylimidazole, arginine, histidine, pyrrolidine, proline, pyrrolidone, pyrrolidone-5-carboxylic acid, pyrazole, 1,2,4-triazole, piperazidine or any mixtures thereof.

**10.** The substance as claimed in one of claims 1 to 9, **characterized in that** it additionally comprises oxidizing agents, in particular $H_2O_2$, in an amount of from 0.01 to 6% by weight, based on the application solution.

**11.** The substance as claimed in one of claims 1 to 10, **characterized in that** it additionally comprises, as oxidation dye precursor, at least one developer component and optionally at least one coupler component.

**12.** The substance as claimed in one of claims 1 to 11, **characterized in that** it additionally comprises at least one direct dye, preferably in an amount of from 0.01 to 20% by weight, based on the total colorant.

**13.** The substance as claimed in one of claims 1 to 12, **characterized in that** it additionally comprises anionic, zwitterionic or nonionic surfactants.

**14.** The use of at least one compound according to formula I according to claim 1 in combination with at least one compound of component B according to claim 1 as a dyeing component in hair colorants.

**15.** A method of dyeing keratinic fibers, in particular human hair, in which a colorant according to one of claims 1 to 13, and customary cosmetic ingredients, is applied to the keratinic fibers, left on the fibers for a certain time, usually about 15-30 minutes, and is then rinsed out again or washed out with a shampoo.

**16.** The method as claimed in claim 15, **characterized in that**, in a two-step method, before or after application of the compound according to formula I according to claim 1, component B is applied to the keratinic fibers, the mixture obtained on the hair is left on the fibers for a certain time, usually about 15-30 minutes, and is then rinsed out again or washed out with a shampoo.

**17.** The method as claimed in one of claims 15 or 16, **characterized in that**, before a colorant according to one of claims 1 to 13 is applied, the keratinic fibers have been bleached in the course of a pretreatment with a bleaching agent or have been colored with an oxidation colorant.

**18.** The use of at least one compound according to formula I and/or its enamine form,

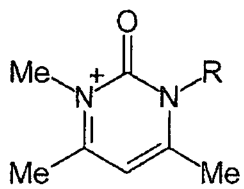

$X^-$ (I)

where

- R is an allyl group, a hydroxy-($C_2$- to $C_6$)-alkyl group or an optionally substituted benzyl group,
- $X^-$ is a physiologically compatible anion

together with at least one aldehyde as component B chosen from the group consisting of 4-hydroxy-3-methoxybenzaldehyde, 3,5-dimethoxy-4-hydroxybenzaldehyde, 4-hydroxy-1-naphthaldehyde, 4-hydroxy-2-methoxybenzaldehyde, 3,4-dihydroxy-5-methoxybenzaldehyde, 3,4,5-trihydroxybenzaldehyde, 3,5-dibromo-4-hydroxybenzaldehyde, 4-hydroxy-3-nitrobenzaldehyde, 3-bromo-4-hydroxybenzaldehyde, 4-hydroxy-3-methylbenzaldehyde, 3,5-dimethyl-4-hydroxybenzaldehyde, 5-bromo-4-hydroxy-3-methoxybenzaldehyde, 4-diethylamino-2-hydroxybenzaldehyde and 4-dimethylamino-2-methoxybenzaldehyde, for the nuancing of oxidation colorations of keratinic fibers, in particular human hair.

**19.** The use of at least one compound according to formula I and/or its enamine form,

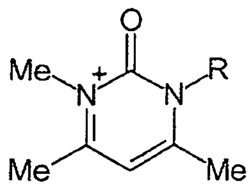

$X^-$ (I)

where

- R is an allyl group, a hydroxy-($C_2$- to $C_6$)-alkyl group or an optionally substituted benzyl group,
- $X^-$ is a physiologically compatible anion

together with at least one aldehyde as component B chosen from the group consisting of 4-hydroxy-3-methoxybenzaldehyde, 3,5-dimethoxy-4-hydroxybenzaldehyde, 4-hydroxy-1-naphthaldehyde, 4-hydroxy-2-methoxybenzaldehyde, 3,4-dihydroxy-5-methoxybenzaldehyde, 3,4,5-trihydroxybenzaldehyde, 3,5-dibromo-4-hydroxybenzaldehyde, 4-hydroxy-3-nitrobenzaldehyde, 3-bromo-4-hydroxybenzaldehyde, 4-hydroxy-3-methylbenzaldehyde, 3,5-dimethyl-4-hydroxybenzaldehyde, 5-bromo-4-hydroxy-3-methoxybenzaldehyde, 4-diethylamino-2-hydroxybenzaldehyde and 4-dimethylamino-2-methoxybenzaldehyde,
for freshening up the color of keratinic fibers colored using oxidative colorants.

**20.** Compounds according to formula I,

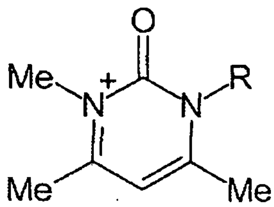

$X^-$ (I)

where

- R is an allyl group, a hydroxy-($C_2$- to $C_6$)-alkyl group or an optionally substituted benzyl group and
- $X^-$ is a physiologically compatible anion.

## Revendications

**1.** Agent pour la teinture de fibres kératiniques, en particulier de cheveux, contenant comme composant A au moins un composé selon la formule I et/ou sa forme énamine,

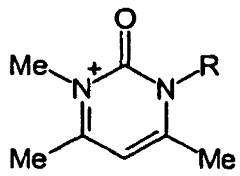

$X^-$ (I)

où

- R représente un groupe allyle, un groupe hydroxy-($C_2$ à $C_6$)-alkyle ou un groupe benzyle le cas échéant substitué,
- $X^-$ signifie un anion physiologiquement acceptable

et comme composant B au moins un aldéhyde choisi dans le groupe constitué par le 4-hydroxy-3-méthoxybenzaldéhyde, le 3,5-diméthoxy-4-hydroxybenzaldéhyde, le 4-hydroxy-1-naphtaldéhyde, le 4-hydroxy-2-méthoxybenzaldéhyde, le 3,4-dihydroxy-5-méthoxybenzaldéhyde, le 3,4,5-trihydroxybenzaldéhyde, le 3,5-dibromo-4-hydroxybenzaldéhyde, le 4-hydroxy-3-nitrobenzaldéhyde, le 3-bromo-4-hydroxybenzaldéhyde, le 4-hydroxy-3-méthylbenzaldéhyde, le 3,5-diméthyl-4-hydroxybenzaldéhyde, le 5-bromo-4-hydroxy-3-méthoxybenzaldéhyde, le 4-diéthylamino-2-hydroxybenzaldéhyde et le 4-diméthylamino-2-méthoxybenzaldéhyde.

2. Agent selon la revendication 1, **caractérisé en ce que** R selon la formule (I) représente un groupe allyle, un groupe 3-hydroxypropyle, un groupe 2-hydroxypropyle, un groupe 2-hydroxyéthyle ou un groupe benzyle.

3. Agent selon l'une quelconque des revendications 1 à 2, **caractérisé en ce que** $X^-$ représente un halogénure, benzènesulfonate, p-toluènesulfonate, ($C_1$ à $C_4$)-alcanesulfonate, trifluorométhanesulfonate, perchlorate, 0,5 sulfate, hydrogénosulfate, tétrafluoroborate, hexafluorophosphate ou tétrachlorozincate.

4. Agent selon l'une quelconque des revendications 1 à 3, **caractérisé en ce que** le composé de formule (I) est choisi dans le groupe constitué par les sels physiologiquement acceptables du 1-allyl-1,2-dihydro-3,4,6-triméthyl-2-oxopyrimidinium, du 1,2-dihydro-1-(2-hydroxyéthyl)-3,4,6-triméthyl-2-oxopyrimidinium, du 1,2-dihydro-1-(3-hydroxypropyl)-3,4,6-triméthyl-2-oxopyrimidinium et du 1-benzyl-1,2-dihydro-3,4,6-triméthyl-2-oxopyrimidinium et les formes énamine des sels susmentionnés.

5. Agent selon l'une quelconque des revendications 1 à 4, **caractérisé en ce qu'**il contient en outre au moins un composé comme composant C, choisi parmi (a) les composés CH-acides, qui sont différents des composés de formule (I) selon la revendication 1, et/ou (b) les composés à groupe carbonyle réactif, qui sont différents des composés du composant B selon la revendication 1.

6. Agent selon la revendication 5, **caractérisé en ce que** les composés CH-acides du composant C sont choisis dans le groupe constitué par des sels formés avec des anions physiologiquement acceptables du 1,4-diméthylquinoléinium, du 1-éthyl-4-méthylquinoléinium, du 1-éthyl-2-méthylquinoléinium, du 1,2,3,3-tétraméthyl-3H-indolium, du 2,3-diméthylbenzothiazolium, du 2,3-diméthylnaphto[1,2-d]thiazolium, du 3-éthyl-2-méthylnaphto[1,2-d]thiazolium, du 3-éthyl-2-méthylbenzoxazolium, du 1,2,3-triméthylquinoxalinium, du 3-éthyl-2-méthylbenzothiazolium, du 1,2-dihydro-1,3,4,6-tétraméthyl-2-oxopyrimidinium, du 1,2-dihydro-1,3,4-triméthyl-2-oxopyrimidinium, du 1,2-dihydro-4,6-diméthyl-1,3-dipropyl-2-oxopyrimidinium, du 1,2-dihydro-1,3,4,6-tétraméthyl-2-thioxopyrimidinium, du 1,2-dihydro-1,3,4,5,6-pentaméthyl-2-oxopyrimidinium, du 2,5-diméthyl-3-(2-propényl)-1,3,4-thiadiazolium, du 3-éthyl-2,5-diméthyl-1,3,4-thiadiazolium, du 1,2-diméthylquinoléinium et de la 1,3,3-triméthyl-2-méthylèneindoline (base de Fischer), de l'oxindole, de la 3-méthyl-1-phénylpyrazolin-5-one, de l'indane-1,2-dione, de l'indane-1,3-dione, de l'indan-1-one, du chlorhydrate de 2-amino-4-imino-1,3-thiazoline, du benzoylacétonitrile, de la 3-dicyanométhylèneindan-1-one, du 2-(2-furannoyl)acétonitrile, du 2-(2-théonyl)acétonitrile, du 2-(cyanométhyl)benzimidazole, du 2-(cyanométhyl)-benzothiazole et du 2-(2,5-diméthyl-3-furannoyl)acétonitrile.

7. Agent selon l'une quelconque des revendications 5 à 6, **caractérisé en ce que** les composés à groupe carbonyle réactif du composant C sont choisis dans le groupe constitué par le coniférylaldéhyde, le 2-méthoxybenzaldéhyde, le 3-méthoxybenzaldéhyde, le 4-méthoxybenzaldéhyde, le 2-éthoxybenzaldéhyde, le 3-éthoxybenzaldéhyde, le 4-éthoxybenzaldéhyde, le 4-hydroxy-2,3-diméthoxybenzaldéhyde, le 4-hydroxy-2,5-diméthoxybenzaldéhyde, le 4-hydroxy-2,6-diméthoxybenzaldéhyde, le 4-hydroxy-2-méthylbenzaldéhyde, le 4-hydroxy-2,3-diméthylbenzaldéhyde, le 4-hydroxy-2,5-diméthylbenzaldéhyde, le 4-hydroxy-2,6-diméthylbenzaldéhyde, le 3,5-diéthoxy-4-hydroxybenzaldéhyde, le 2,6-diéthoxy-4-hydroxybenzaldéhyde, le 3-hydroxy-4-méthoxybenzaldéhyde, le 2-hydroxy-4-méthoxybenzaldéhyde, le 2-éthoxy-4-hydroxybenzaldéhyde, le 3-éthoxy-4-hydroxybenzaldéhyde, le 4-éthoxy-2-hydroxybenzaldéhyde, le 4-éthoxy-3-hydroxybenzaldéhyde, le 2,3-diméthoxybenzaldéhyde, le 2,4-diméthoxybenzaldéhyde, le 2,5-diméthoxybenzaldéhyde, le 2,6-diméthoxybenzaldéhyde, le 3,4-diméthoxybenzaldéhyde, le 3,5-diméthoxybenzaldéhyde, le 2,3,4-triméthoxybenzaldéhyde, le 2,3,5-triméthoxybenzaldéhyde, le 2,3,6-triméthoxybenzaldéhyde, le 2,4,6-triméthoxybenzaldéhyde, le 2,4,5-triméthoxybenzaldéhyde, le 2,5,6-triméthoxybenzaldéhyde, le 2-hydroxybenzaldéhyde, le 3-hydroxybenzaldéhyde, le 4-hydroxybenzaldéhyde, le 2,3-dihydroxybenzaldéhyde, le 2,4-dihydroxybenzaldéhyde, le 2,4-dihydroxy-3-méthylbenzaldéhyde, le 2,4-dihydroxy-5-méthylbenzaldéhyde, le 2,4-dihydroxy-6-méthylbenzaldéhyde, le 2,4-dihydroxy-3-méthoxybenzaldéhyde, le 2,4-dihydroxy-5-méthoxybenzaldéhyde, le 2,4-dihydroxy-6-méthoxybenzaldéhyde, le 2,5-dihydroxybenzaldéhyde, le 2,6-dihydroxybenzaldéhyde, le 3,4-dihydroxybenzaldéhyde, le 3,4-dihydroxy-2-méthylbenzaldéhyde, le 3,4-dihydroxy-5-méthylbenzaldéhyde, le 3,4-dihydroxy-6-méthylbenzaldéhyde, le 3,4-dihydroxy-2-méthoxybenzaldéhyde, le 3,5-dihydroxybenzaldéhyde, le 2,3,4-trihydroxybenzaldéhyde, le 2,3,5-trihydroxybenzaldéhyde, le 2,3,6-trihydroxybenzaldéhyde, le 2,4,6-trihydroxybenzaldéhyde, le 2,4,5-trihydroxybenzaldéhyde, le 2,5,6-trihydroxybenzaldéhyde, le 4-diméthylaminobenzaldéhyde, le 4-diéthylaminobenzaldéhyde, le 4-diméthylamino-2-hydroxybenzaldéhyde, le 4-pyrrolidinobenzaldéhyde, le 4-morpholinobenzaldéhyde, le 2-morpholinobenzaldéhyde, le 4-pipéridinobenzaldéhyde, le 3,5-dichloro-4-hydroxybenzaldéhyde, le 4-hydroxy-3,5-diiodobenzaldéhyde, le 3-chloro-4-hydroxybenzaldéhyde, le 5-chloro-3,4-dihydroxybenzaldéhyde, le 5-bromo-3,4-dihydroxybenzaldéhyde, le 3-chloro-4-hydroxy-5-méthoxybenzaldéhyde, le 4-hydroxy-3-iodo-5-méthoxybenzaldéhyde, le 2-méthoxy-1-naphtaldéhyde, le 4-méthoxy-1-naphtaldéhyde, le 2-hydroxy-1-naphtaldéhyde, le 2,4-dihydroxy-1-napthaldéhyde, le 4-hydroxy-3-méthoxy-1-naphtaldéhyde, le 2-hydroxy-4-méthoxy-1-naphtaldéhyde, le 3-hydroxy-4-méthoxy-1-naphtaldéhyde, le 2,4-diméthoxy-1-naphtaldéhyde, le 3,4-diméthoxy-1-naphtaldéhyde, le 4-diméthylamino-1-naphtaldéhyde, le 2-nitrobenzaldéhyde, le 3-nitrobenzaldéhyde, le 4-nitrobenzaldéhyde, le 4-méthyl-3-nitrobenzaldéhyde, le 3-hydroxy-4-nitrobenzaldéhyde, le 5-hydroxy-2-nitrobenzaldéhyde, le 2-hydroxy-5-nitrobenzaldéhyde, le 2-hydroxy-3-nitrobenzaldéhyde, le 2-fluoro-3-nitrobenzaldéhyde, le 3-méthoxy-2-nitrobenzaldéhyde, le 4-chloro-3-nitrobenzaldéhyde, le 2-chloro-6-nitrobenzaldéhyde, le 5-chloro-2-nitrobenzaldéhyde, le 4-chloro-2-nitrobenzaldéhyde, le 2,4-dinitrobenzaldéhyde, le 2,6-dinitrobenzaldéhyde, le 2-hydroxy-3-méthoxy-5-nitrobenzaldéhyde, le 4,5-diméthoxy-2-nitrobenzaldéhyde, le 6-nitropipéronal, le 2-nitropipéronal, la 5-nitrovanilline, l'aldéhyde 2,5-dinitrosalicylique, l'aldéhyde 5-bromo-3-nitrosalicylique, le 4-nitro-1-naphtaldéhyde, l'aldéhyde 2-nitrocinnamique, l'aldéhyde 3-nitrocinnamique, l'aldéhyde 4-nitrocinnamique, l'aldéhyde 4-diméthylaminocinnamique, le 2-diméthylaminobenzaldéhyde, le 2-chloro-4-diméthylaminobenzaldéhyde, le 4-diméthylamino-2-méthylbenzaldéhyde, l'aldéhyde 4-diéthylaminocinnamique, le 4-dibutylaminobenzaldéhyde, le 4-diphénylaminobenzaldéhyde, le 4-(1-imidazolyl)-benzaldéhyde et le pipéronal.

8. Agent selon l'une quelconque des revendications 1 à 7, **caractérisé en ce que** les composés de formule I, les composés du composant B et les composés du composant C sont à chaque fois contenus en une quantité de 0,03 à 65 mmoles, en particulier de 1 à 40 mmoles, par rapport à 100 g de la totalité de l'agent de teinture.

9. Agent selon l'une quelconque des revendications 1 à 8, **caractérisé en ce qu'**il contient un agent de renforcement de la couleur choisi dans le groupe formé par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'arginine, l'histidine, la pyrrolidine, la proline, la pyrrolidone, l'acide pyrrolidone-5-carboxylique, le pyrazole, le 1,2,4-triazole, la pipérazidine ou leurs mélanges quelconques.

10. Agent selon l'une quelconque des revendications 1 à 9, **caractérisé en ce qu'**il contient en outre des oxydants, en particulier $H_2O_2$ en une quantité de 0,01 à 6% en poids, par rapport à la solution d'utilisation.

11. Agent selon l'une quelconque des revendications 1 à 10, **caractérisé en ce qu'**il contient en outre, comme précurseur d'un colorant d'oxydation, au moins un composant de développement et le cas échéant au moins un composant de couplage.

12. Agent selon l'une quelconque des revendications 1 à 11, **caractérisé en ce qu'**il contient en outre au moins un colorant montant directement sur la fibre, de préférence en une quantité de 0,01 à 20% en poids, par rapport à la totalité de l'agent de teinture.

13. Agent selon l'une quelconque des revendications 1 à 12, **caractérisé en ce qu'**il contient en outre des agents tensioactifs anioniques, zwittérioniques ou non ioniques.

**14.** Utilisation d'au moins un composé selon la formule I selon la revendication 1, en combinaison avec au moins un composé du composant B selon la revendication 1, comme composant de teinture dans des agents de teinture pour cheveux.

**15.** Procédé pour la teinture de fibres kératiniques, en particulier de cheveux, dans lequel un agent de teinture selon l'une quelconque des revendications 1 à 13, ainsi que des constituants cosmétiques usuels sont appliqués sur les fibres kératiniques, on les laisse pendant un certain laps de temps, usuellement d'environ 15-30 minutes sur les fibres, puis on les élimine par rinçage ou par lavage avec un shampooing.

**16.** Procédé selon la revendication 15, **caractérisé en ce qu'**on applique, dans un procédé à deux étapes, le composant B selon la revendication 1 avant ou après l'application du composé selon la formule I selon la revendication 1 sur les fibres kératiniques, on laisse le mélange obtenu sur les cheveux pendant un certain laps de temps, usuellement d'environ 15-30 minutes, sur les fibres, puis on l'élimine par rinçage ou par lavage avec un shampooing.

**17.** Procédé selon l'une quelconque des revendications 15 ou 16, **caractérisé en ce que** les fibres kératiniques, avant l'utilisation d'un agent de teinture selon l'une quelconque des revendications 1 à 13, dans le cadre d'un prétraitement, sont éclaircies avec un agent de décoloration ou teintes avec un agent de teinture par oxydation.

**18.** Utilisation d'au moins un composé selon la formule I et/ou sa forme énamine,

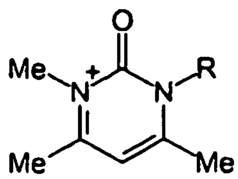

$X^-$ (I)

où

- R représente un groupe allyle, un groupe hydroxy-($C_2$ à $C_6$)-alkyle ou un groupe benzyle le cas échéant substitué,
- $X^-$ signifie un anion physiologiquement acceptable

avec au moins un aldéhyde comme composant B choisi dans le groupe constitué par le 4-hydroxy-3-méthoxybenzaldéhyde, le 3,5-diméthoxy-4-hydroxybenzaldéhyde, le 4-hydroxy-1-naphtaldéhyde, le 4-hydroxy-2-méthoxybenzaldéhyde, le 3,4-dihydroxy-5-méthoxybenzaldéhyde, le 3,4,5-trihydroxybenzaldéhyde, le 3,5-dibromo-4-hydroxybenzaldéhyde, le 4-hydroxy-3-nitrobenzaldéhyde, le 3-bromo-4-hydroxybenzaldéhyde, le 4-hydroxy-3-méthylbenzaldéhyde, le 3,5-diméthyl-4-hydroxybenzaldéhyde, le 5-bromo-4-hydroxy-3-méthoxybenzaldéhyde, le 4-diéthylamino-2-hydroxybenzaldéhyde et le 4-diméthylamino-2-méthoxybenzaldéhyde pour nuancer les teintures d'oxydation de fibres kératiniques, en particulier les cheveux humains.

**19.** Utilisation d'au moins un composé selon la formule I et/ou sa forme énamine,

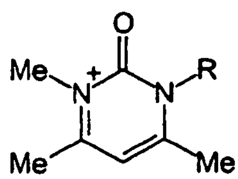

$X^-$ (I)

où

- R représente un groupe allyle, un groupe hydroxy-($C_2$ à $C_6$)-alkyle ou un groupe benzyle le cas échéant substitué,
- $X^-$ signifie un anion physiologiquement acceptable

et avec au moins un aldéhyde comme composant B choisi dans le groupe constitué par le 4-hydroxy-3-méthoxybenzaldéhyde, le 3,5-diméthoxy-4-hydroxybenzaldéhyde, le 4-hydroxy-1-naphtaldéhyde, le 4-hydroxy-2-méthoxybenzaldéhyde, le 3,4-dihydroxy-5-méthoxybenzaldéhyde, le 3,4,5-trihydroxybenzaldéhyde, le 3,5-dibromo-4-hydroxybenzaldéhyde, le 4-hydroxy-3-nitrobenzaldéhyde, le 3-bromo-4-hydroxybenzaldéhyde, le 4-hydroxy-3-méthylbenzaldéhyde, le 3,5-diméthyl-4-hydroxybenzaldéhyde, le 5-bromo-4-hydroxy-3-méthoxybenzaldéhyde, le 4-diéthylamino-2-hydroxybenzaldéhyde et le 4-diméthylamino-2-méthoxybenzaldéhyde pour rafraîchir la teinte de fibres kératiniques teintes avec des agents de teinture par oxydation.

**20.** Composés selon la formule I,

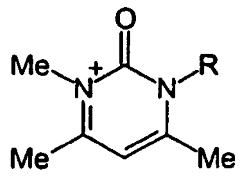

$X^-$ (I)

où

- R représente un groupe allyle, un groupe hydroxy-($C_2$ à $C_6$)-alkyle ou un groupe benzyle le cas échéant substitué, et
- $X^-$ signifie un anion physiologiquement acceptable.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2047431 A1
- DE 2165913 A1
- DE 10241076 A1
- EP 998908 A2
- JP 2002047153 A
- GB 1026978 A
- GB 1153196 A
- DE 2359399
- JP 02019576 A
- WO 9615765 A
- DE 3843892
- DE 4133957
- WO 9408969 A
- WO 9408970 A
- EP 740931 A
- DE 19543988
- DE 3725030 A
- DE 3723354 A
- DE 3926344 A


### In der Beschreibung aufgeführte Nicht-Patentliteratur


- **VON CH. CULNAN ; H. MAIBACH.** Dermatology. Verlag Marcel Dekker Inc, 1986, vol. 7
- **CH. ZVIAK.** Europäische Inventar der Kosmetikrohstoffe. *The Science of Hair Care,* 1996, 248-250264-267
- **H. BAUMANN et al.** *Liebigs Ann. Chem.,* 1968, vol. 717, 124-136
- **D. LLOYD et al.** *J. Chem. Soc. Perkin Trans I,* 1977, vol. 16, 1862-1869
- **S. T. OSWALD et al.** *J. Heterocycl. Chem.,* 1974, vol. 11 (3), 441-443
- **V. A. CHUIGUK.** *Ukr. Khim. Zh. (Russ Ed.),* 1982, vol. 48 (11), 1220-1223
- **H. BAUMANN et al.** *J. Liebigs Ann. Chem.,* 1968, vol. 717, 124-136
- **WILLIAM J. HALE.** *J. Am. Chem. Soc.,* 1914, vol. 36, 104-115
- **V. S. REZNIK.** *Pharmaceutical Chemistry Journal (Translation of Khimiko-Farmatsevticheskü Zhurnal),* 2001, vol. 35 (12), 672-676